# EUROPÄISCHE PATENTSCHRIFT

(11) **EP 4 065 560 B1**
(45) Veröffentlichungstag und Bekanntmachung des Hinweises auf die Patenterteilung: **13.09.2023**
(21) Anmeldenummer: 20726862.4
(22) Anmeldetag: 26.05.2020
(51) Int. Cl.: C07D 211/94

(54) **VERBESSERTES VERFAHREN ZUR HERSTELLUNG WÄSSRIGER LÖSUNGEN VON 4-AMMONIUM-ALKYLPIPERIDIN-1-YLOXYLSALZEN ZUM EINSATZ IN LADUNGSSPEICHEREINHEITEN**
IMPROVED METHOD FOR PREPARING AQUEOUS SOLUTIONS OF 4-AMMONIUM ALKYLPIPERIDINE-1-YLOXYL SALTS FOR USE IN CHARGE STORAGE UNITS
PROCÉDÉ AMÉLIORÉ DE FABRICATION DE SOLUTIONS AQUEUSES DES SELS DE 4-AMMONIUM-ALKYLPIPÉRIDINE-1-YLOXYLE DESTINÉS À L'UTILISATION DANS DES UNITÉS D'ACCUMULATEUR DE CHARGE

(30) Priorität: 29.11.2019 EP 19212511
(43) Veröffentlichungstag der Anmeldung: 05.10.2022
(73) Patentinhaber: Evonik Operations GmbH, 45128 Essen (DE)
(72) Erfinder: GÄRTNER, Felix, 45721 Haltern am See (DE); NISSEN, Felix, 48301 Nottuln (DE)
(74) Vertreter: Evonik Patent Association
(86) Internationale Anmeldenummer: PCT/EP2020/064493
(87) Internationale Veröffentlichungsnummer: WO 2021/104684

(56) Entgegenhaltungen:
- DE-A1-102016 009 904
- US-A1- 2018 072 669
- TOBIAS JANOSCHKA ET AL: "An Aqueous Redox-Flow Battery with High Capacity and Power: The TEMPTMA/MV System", ANGEWANDTE CHEMIE, INTERNATIONAL EDITION, Bd. 55, Nr. 46, 18. Oktober 2016 (2016-10-18), Seiten 14427-14430, XP055494176, DE ISSN: 1433-7851, DOI: 10.1002/anie.201606472
- JIAN LUO ET AL: "A [pi]-Conjugation Extended Viologen as a Two-Electron Storage Anolyte for Total Organic Aqueous Redox Flow Batteries", ANGEWANDTE CHEMIE, INTERNATIONAL EDITION, Bd. 57, Nr. 1, 13. Dezember 2017 (2017-12-13), Seiten 231-235, XP055691320, DE ISSN: 1433-7851, DOI: 10.1002/anie.201710517

## Beschreibung

Die vorliegende Erfindung betrifft ein Verfahren zur Herstellung wässriger Lösungen von 4-Ammonium-alkylpiperidin-1-yloxylsalzen. Darin erfolgt eine Umsetzung des Eduktes, eines 4-Alkylammoniumsalzes des 2,2,6,6-Tetramethylpiperidins, mit H₂O₂ in wässriger Lösung in Gegenwart mindestens eines Salzes ausgewählt aus Hydrogencarbonatsalz, Carbonatsalz. Das erfindungsgemäße Verfahren zeichnet sich durch den sparsamen Einsatz der Edukte aus.

### Hintergrund der Erfindung

Die Synthese von 4-Ammonium-alkylpiperidin-1-yloxysalzen ist von großer Bedeutung für das Gebiet der organischen Batterien, in welchen solche Verbindungen als Elektrolytmaterial eingesetzt werden. Die Synthese solcher Materialien ist vielfach im Stand der Technik beschrieben. Der übliche Syntheseweg verläuft dabei über zwei Stufen:
Stufe **1**: 4-Aminoalkylpiperidin wird mit Alkylhalogenid oder einem anderen Alkylierungsmittel zum 4-Ammonium-alkylpiperidinylsalz umgesetzt.
Stufe **2**: Das 4-Ammonium-alkylpiperidinylsalz wird durch Oxidation mit einer Perverbindung wie beispielsweise H₂O₂ zum 4-Ammonium-alkylpiperidin-1-yloxysalz umgesetzt.

In der Stufe **1** wird üblicherweise das entsprechende Alkyljodid eingesetzt, wie etwa von C. Francavilla et al., Bioorganic and Medicinal Chemistry Letters 2011, 21, 3029-3033 auf Seite 3031 in Schema 6 bei der Umsetzung von Verbindung **38** in Verbindung **39** beschrieben. Die gleiche Reaktion wird auch in Janoschka et al., Angew. Chem. Int. Ed. 2016, 55, 14427-14430 (im Folgenden "Janoschka et *al*.") auf Seite 14429 in Schema 1 bei der Umsetzung von Verbindung **2** in Verbindung **3** beschrieben.

B. Bales et al., J. Phys. Chem. A 2009, 113, 9295-9303 beschreiben auf Seite 9298, Schema 1, die Umsetzung eines deuterierten 4-Aminoalkylpiperidinderivats mit n-Dodecylbromid zum entsprechenden Bromidsalz.

B. K. Sinha & C. F. Chignell, J. Med. Chem. 1975, 18, 669-673 (im Folgenden "Sinha & Chignell") beschreiben die entsprechende Umsetzung von 4-Dimethylamino-2,2,6,6-tetramethylpiperidin mit 1,6-Dibromhexan bzw. 1,10-Dibromdecan zum jeweiligen Bromidsalz.

Sowohl Janoschka et *al.* als auch Sinha & Chignell beschreiben außerdem die weitere Stufe (Stufe **2**), also die Oxidation der sekundären Aminogruppe zum jeweiligen N-O·-Radikal. Als Oxidationsmittel werden H₂O₂ und Natriumwolframat eingesetzt.

Auch US2018/0072669 und DE102016009904 offenbaren die genannte Oxidation mit H₂O₂ und Natriumwolframat. Als Alternative offenbart DE102016009904 zudem auch die Oxidation mit H₂O₂ und Magnesiumsulfat.

JPH08-3136 und RU 2114830 C1 behandeln die Überführung nichtionischer 4-Oxo- oder 4-OH-Derivate des 2,2,6,6-Tetramethylpiperidins in das entsprechende Nitroxyl-Radikal mittels Umsetzung mit H₂O₂ in Gegenwart von CO₂.

Die im Stand der Technik beschriebenen Verfahren, welche der oben genannten Stufe **1** entsprechen, wenden üblicherweise Alkylbromid oder Alkyljodid als Alkylierungsmittel an.

Dies ist insofern von Nachteil, als in den Fällen, in denen ein Chloridsalz gewünscht ist, ein Anionenaustausch des Jodids oder Bromids durch Chlorid erfolgen muss, was einen zusätzlichen Schritt erfordert und die gesamte Synthese somit aufwendiger macht.

Dieser Nachteil kann durch die direkte Umsetzung des 4-Aminopiperidins mit Alkylchloriden und Sulfaten, wie in der WO 2018/028830 A1 und von Janoschka et *al*. beschrieben, umgangen werden.

In diesen Offenbarungen erfolgt im Einzelnen eine direkte Umsetzung mit Methylchlorid. Der Einsatz von Methylchlorid hat allerdings wiederum den Nachteil, dass Methylchlorid weniger reaktiv ist als das entsprechende Methyljodid oder Methylbromid und die Reaktion somit langsamer verläuft. Ein weiterer Nachteil der von WO 2018/028830 A1 und von Janoschka et *al.* beschriebenen Synthese ist außerdem, dass die Stufe **2**, also die Oxidation zum entsprechenden N-O·-Radikal, unter Verwendung von Erdalkalisulfaten mit einem großen Überschuss an H₂O₂ erfolgt.

Diese Reaktionsführung ist schon allein aufgrund des hohen Ressourcenverbrauchs nachteilig. Daneben wurde beobachtet, dass die im Stand der Technik beschriebene Oxidation zu Niederschlägen führt, welche die Aufarbeitung *per se* verkomplizieren.

Die erfindungsgemäße Aufgabe besteht demnach darin, ein Verfahren Herstellung von 4-Ammonium-alkylpiperidin-1-yloxylsalzen zur Verfügung zu stellen, welches die beschriebenen Nachteile der herkömmlichen Verfahren nicht aufweist.

Es wurde nun überraschend ein Verfahren gefunden, welches die erfindungsgemäße Aufgabe löst.

### Detaillierte Beschreibung der Erfindung

Die vorliegende Erfindung betrifft demnach ein Verfahren zur Herstellung einer wässrigen Elektrolytlösung **L₁** umfassend mindestens eine Verbindung der Formel **(I)** oder **(II),** bevorzugt mindestens eine Verbindung der Formel **(I),** mit 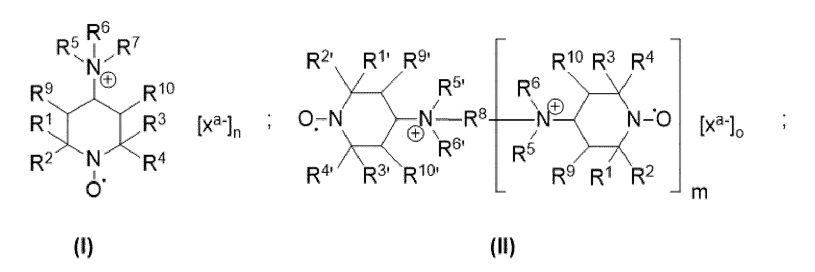
wobei R¹, R², R³, R⁴, R¹', R²', R³', R⁴' unabhängig voneinander jeweils ein C₁-C₆-Alkylrest, insbesondere ein C₁-C₄-Alkylrest, bevorzugt ein C₁-C₂-Alkylrest, und bevorzugter alle jeweils Methyl sind,
wobei R⁹, R¹⁰, R⁹', R¹⁰' unabhängig voneinander jeweils aus der Gruppe bestehend aus Wasserstoff, C₁-C₆-Alkylrest, insbesondere aus der Gruppe bestehend aus Wasserstoff, C₁-C₄-Alkylrest, bevorzugt aus der Gruppe bestehend aus Wasserstoff, Methyl, Ethyl ausgewählt sind, und bevorzugter alle Wasserstoff sind,
wobei R⁵, R⁶, R⁵', R⁶' unabhängig voneinander jeweils aus der Gruppe bestehend aus Wasserstoff, Alkyl, Cycloalkyl, Aryl, Aralkyl, Heterocyclyl ausgewählt sind, wobei die Reste Alkyl, Cycloalkyl, Aryl, Aralkyl, Heterocyclyl mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid, Halogen substituiert sein können, wobei außerdem Alkyl durch ein oder mehrere, nicht direkt zueinander benachbarte zweiwertige Reste ausgewählt aus der Gruppe bestehend aus Sauerstoff, Imino unterbrochen sein kann,
wobei außerdem R⁵ und R⁶ zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei außerdem R⁵' und R⁶' zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei insbesondere R⁵, R⁶, R⁵', R⁶' unabhängig voneinander jeweils aus der Gruppe bestehend aus Wasserstoff, Alkyl, wobei Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, wobei außerdem R⁵ und R⁶ eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können, und wobei außerdem R⁵' und R⁶' zusammen eine Alkylengruppe die durch ein oder mehrere, nicht direkt zueinander benachbarte zweiwertige Reste ausgewählt aus der Gruppe bestehend aus Sauerstoff, Imino unterbrochen sein kann, bilden können,
wobei bevorzugt R⁵, R⁶, R⁵', R⁶' unabhängig voneinander jeweils aus der Gruppe bestehend aus C₁-C₆-Alkyl, wobei C₁-C₆-Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, wobei außerdem R⁵ und R⁶ eine C₁-C₁₀-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können, und wobei außerdem R⁵' und R⁶' zusammen eine C₁-C₁₀-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei noch bevorzugter R⁵, R⁶, R⁵', R⁶' unabhängig voneinander jeweils eine C₁-C₅-Alkylgruppe, die durch ein oder mehrere, nicht direkt zueinander benachbarte zweiwertige Reste ausgewählt aus der Gruppe bestehend aus Sauerstoff, Imino unterbrochen sein kann, sind, wobei außerdem R⁵ und R⁶ eine C₁-C₆-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können, wobei außerdem R⁵' und R⁶' zusammen eine C₁-C₆-Alkylengruppe die durch ein oder mehrere, nicht direkt zueinander benachbarte zweiwertige Reste ausgewählt aus der Gruppe bestehend aus Sauerstoff, Imino unterbrochen sein kann, bilden können,
wobei noch mehr bevorzugter R⁵, R⁶, R⁵', R⁶' unabhängig voneinander jeweils aus der Gruppe bestehend aus Methyl, Ethyl, *n*-Propyl, *n*-Butyl, -CH₂CH₂(NH)CH₃, -CH₂CH₂(NCH₃)CH₂CH₂(NH)CH₃ ausgewählt sind, wobei außerdem R⁵ und R⁶ zusammen eine Gruppe ausgewählt aus Piperidin, Morpholin, Piperazin, Pyrrolidin bilden können und wobei außerdem R⁵' und R⁶' zusammen eine Gruppe ausgewählt aus Piperidin, Morpholin, Piperazin, Pyrrolidin bilden können,
wobei noch viel mehr bevorzugter R⁵, R⁶, R⁵', R⁶' unabhängig voneinander jeweils aus der Gruppe bestehend aus Methyl, Ethyl, *n*-Propyl ausgewählt sind und am allerbevorzugtesten R⁵, R⁶, R⁵', R⁶' jeweils Methyl sind,
wobei R⁷ ein Alkylrest ist, der mit ein oder mehreren Resten ausgewählt aus der Gruppe bestehend aus Alkoxy, Hydroxy, Nitro, Carboxyl, Carboxylester, Carboxylamid, Sulfonat, Sulfonsäureester, Sulfonamid, Halogen substituiert sein kann und der durch ein oder mehrere, nicht direkt zueinander benachbarte zweiwertige Reste ausgewählt aus der Gruppe bestehend aus Sauerstoff, Imino unterbrochen sein kann,
wobei R⁷ insbesondere C₁-C₆-Alkyl, welches durch ein oder mehrere, nicht direkt zueinander benachbarte zweiwertige Reste ausgewählt aus der Gruppe bestehend aus Sauerstoff, Imino unterbrochen sein kann, ist,
wobei R⁷ bevorzugt C₁-C₅-Alkyl, welches durch ein oder mehrere, nicht direkt zueinander benachbarte zweiwertige Reste ausgewählt aus der Gruppe bestehend aus Sauerstoff, Imino unterbrochen sein kann, ist,
wobei R⁷ bevorzugter aus der Gruppe bestehend aus Methyl, Ethyl, *n*-Propyl, *n*-Butyl, -CH₂CH₂(NH)CH₃, -CH₂CH₂(NCH₃)CH₂CH₂(NH)CH₃ ausgewählt ist,
wobei R⁷ noch bevorzugter aus der Gruppe bestehend aus Methyl, Ethyl, *n*-Propyl ausgewählt ist,
wobei R⁷ noch mehr bevorzugter aus der Gruppe bestehend aus Methyl, Ethyl ausgewählt ist,
und R⁷ am bevorzugtesten Methyl ist,
wobei R⁸ eine p-wertige organische Brückengruppe ist, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann, und die durch ein oder mehrere, nicht direkt zueinander benachbarte zweiwertige Reste ausgewählt aus der Gruppe bestehend aus Sauerstoff, Imino unterbrochen sein kann,
wobei R⁸ insbesondere eine Alkylengruppe, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann und die durch ein oder mehrere, nicht direkt zueinander benachbarte zweiwertige Reste ausgewählt aus der Gruppe bestehend aus Sauerstoff, Imino unterbrochen sein kann, ist,
wobei R⁸ bevorzugt eine Alkylengruppe, die durch ein oder mehrere, nicht direkt zueinander benachbarte zweiwertige Reste ausgewählt aus der Gruppe bestehend aus Sauerstoff, Imino unterbrochen sein kann, ist,
wobei R⁸ bevorzugter eine Alkylengruppe ist, noch bevorzugter eine C₁-C₁₀-Alkylengruppe, noch mehr bevorzugter eine C₁-C₆-Alkylengruppe, noch viel mehr bevorzugter eine C₂-C₆-Alkylengruppe, am bevorzugtesten aus der Gruppe bestehend aus Ethylen (also "-CH₂CH₂-), Butylen (also "-CH₂CH₂CH₂CH₂-"), Hexamethylen (also "-CH₂CH₂CH₂CH₂CH₂CH₂-") ausgewählt ist, und am allerbevorzugtesten Hexamethylen ist,
a die Wertigkeit des Anions X^{a-} angibt und eine ganze Zahl von 1 bis 10.000, insbesondere 1 bis 50, bei Verbindungen der Formel **(I)** und **(III)** bevorzugt 1 bis 3, bevorzugter 1 bis 2 und noch bevorzugter 1 bedeutet und bei Verbindungen der Formel **(II)** und **(IV)** bevorzugt 1 bis 6, noch bevorzugter 1 bis 5, noch mehr bevorzugter 1 bis 4, noch mehr bevorzugter 1 bis 3 und am bevorzugtesten 1 bis 2 bedeutet, am allerbevorzugtesten 1 bedeutet,
p eine ganze Zahl von 2 bis 6, insbesondere 2 bis 5, noch bevorzugter 2 bis 4, noch mehr bevorzugter 2 bis 3 und am bevorzugtesten 2 bedeutet,
m eine ganze Zahl mit dem Wert "p - 1" bedeutet,
n eine Zahl mit dem Wert "1 / a" bedeutet,
o eine Zahl mit dem Wert "(1 + m) / a" bedeutet, und
X^{a-} ein organisches Anion, ein anorganisches Anion oder eine Mischung dieser Anionen ist, insbesondere ein anorganisches Anion ist, wobei X^{a-} bevorzugt aus der Gruppe bestehend aus Halogenidionen (Wertigkeit a = 1), Hydroxyionen (Wertigkeit a = 1), Phosphationen (Wertigkeit a = 3), Sulfationen (Wertigkeit a = 2), Perchlorationen (Wertigkeit a = 1), Hexafluorophosphationen (Wertigkeit a = 1), Tetrafluoroborationen (Wertigkeit a = 1) ausgewählt ist, bevorzugter aus der Gruppe bestehend aus Halogenidionen, Sulfatanion ausgewählt ist, noch bevorzugter aus der Gruppe bestehend aus Chlorid, Methylsulfat ausgewählt ist,
gekennzeichnet dadurch, dass
   (a) mindestens eine Verbindung der Formel **(III)** oder **(IV)** mit 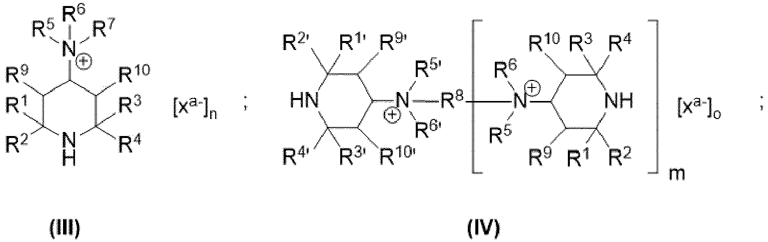 mit H₂O₂ in einer wässrigen Lösung **L₂** umfassend mindestens ein Salz **S,** was aus der Gruppe bestehend aus Hydrogencarbonatsalz, Carbonatsalz ausgewählt ist, zu einer Verbindung der Formel **(I)** bzw. **(II)** umgesetzt wird und
   (b) nach der Umsetzung gemäß Schritt (a) H₂O₂, falls dieses dann noch in der wässrigen Lösung **L₂** enthalten ist, aus **L₂** entfernt wird,
      wodurch eine wässrige Lösung **L₃** umfassend mindestens eine Verbindung der Formel **(I)** bzw. **(II)** und mindestens ein Salz **S** erhalten wird,
   (c) ein saurer pH-Wert in **L₃** eingestellt wird, wodurch das Salz **S** mindestens teilweise aus **L₃** entfernt und die wässrige Elektrolytlösung **L₁** erhalten wird.

In der bevorzugten Ausführungsform, in der im erfindungsgemäßen Verfahren eine wässrigen Lösung **L₁** mindestens einer Verbindung der Formel **(I)** hergestellt wird und deshalb eine Verbindung der Formel **(III)** eingesetzt wird, gelten die vorgenannten Definitionen der Reste R¹, R², R³, R⁴, R⁵, R⁶, R⁷, R⁹, R¹⁰, X^{a-} und der Variablen n entsprechend.

Das erfindungsgemäße Verfahren hat den überraschenden Vorteil, dass keine aufwendige Aufarbeitung wie Filtration der wässrigen Lösung **L₁** durchgeführt werden muss, um zu einem Produkt zu gelangen, welches in Ladungsspeichereinheiten angewendet werden kann. Dies war überraschend im Lichte des Standes der Technik wie JP-H08-3136 und RU 2114830 C1. Weiterhin ermöglicht der Einsatz von Carbonaten und Hydrogencarbonaten, die als Katalysator fungieren, milde Reaktionsbedingungen, so dass die Menge an H₂O₂ relativ zu der in den Verfahren des Standes der Technik eingesetzten Menge, auf einen Bruchteil reduziert werden kann.

Das erfindungsgemäße Verfahren wird bevorzugt kontinuierlich durchgeführt.

### 1. Schritt (a)

In Schritt (a) des erfindungsgemäßen Verfahrens wird mindestens eine Verbindung der Formel **(III)** oder **(IV)** mit H₂O₂ in einer wässrigen Lösung **L₂** umfassend mindestens ein Salz **S,** was aus der Gruppe bestehend aus Hydrogencarbonatsalz, Carbonatsalz ausgewählt ist, zu einer Verbindung der Formel **(I)** bzw. **(II)** umgesetzt. Es wird demnach eine wässrige Lösung **L₂** eingesetzt.

Die wässrige Lösung **L₂** umfasst mindestens eine Verbindung der Formel **(III)** oder **(IV),** bzw. in den Ausführungsformen des erfindungsgemäßen Verfahrens, in denen eine wässrige Lösung **L₁** umfassend mindestens eine Verbindung der Formel **(I)** hergestellt wird, mindestens eine Verbindung der Formel **(III).**

"Zu einer Verbindung der Formel **(I)** bzw. **(II)** umgesetzt wird" ist so zu verstehen, dass, wenn eine Verbindung der Formel **(III)** in Schritt (a) des erfindungsgemäßen Verfahrens eingesetzt wird, dann eine Verbindung der Formel **(I)** als Produkt erhalten wird, und wenn eine Verbindung der Formel **(IV)** in Schritt (a) des erfindungsgemäßen Verfahrens eingesetzt wird, dann eine Verbindung der Formel **(II)** als Produkt erhalten wird.

Insbesondere wird Schritt (a) des erfindungsgemäßen Verfahrens dabei bei einem pH-Wert von 7.5 bis < 10.5, bevorzugt 8 bis < 10.5, bevorzugter 8.5 bis < 10.5, noch bevorzugter 9 bis 10, noch mehr bevorzugter 9.5 bis 10 durchgeführt.

Insbesondere wird Schritt (a) des erfindungsgemäßen Verfahrens dabei bei einer Temperatur von 20 °C bis 100 °C, bevorzugter 30 °C bis 80 °C, noch bevorzugter 40 °C bis 75 °C, am bevorzugtesten 60 °C bis 75 °C durchgeführt.

Insbesondere wird Schritt (a) des erfindungsgemäßen Verfahrens dabei bei einem Druck von 0.5 bar bis 2 bar, bevorzugt bei 1 bar durchgeführt.

Die wässrige Lösung **L₂** umfasst mindestens ein Salz **S** ausgewählt aus der Gruppe bestehend aus Hydrogencarbonatsalz, Carbonatsalz, bevorzugt mindestens ein Hydrogencarbonatsalz.

Als Hydrogencarbonatsalz oder Carbonatsalz können alle entsprechenden Salze eingesetzt werden, die das erfindungsgemäße Verfahren nicht verhindern. In einer vorteilhaften Ausführungsform des erfindungsgemäßen Verfahrens wird das Salz **S** aus der Gruppe bestehend aus Alkalimetallhydrogencarbonat, Erdalkalimetallhydrogencarbonat, Alkalimetallcarbonat, Erdalkalimetallcarbonat ausgewählt, bevorzugter aus der Gruppe bestehend aus Alkalimetallhydrogencarbonat, Erdalkalimetallcarbonat, am bevorzugtesten Alkalimetallhydrogencarbonat. Bevorzugte Alkalimetalle sind Lithium, Kalium, Natrium, noch bevorzugtere Alkalimetalle sind Kalium, Natrium, am bevorzugtesten ist Natrium. Bevorzugte Erdalkalimetalle sind Magnesium, Calcium, am bevorzugtesten ist Magnesium.

Das bevorzugteste Alkalimetallhydrogencarbonat und auch das bevorzugteste Salz **S** ist Natriumhydrogencarbonat.

In einer bevorzugten Ausführungsform des erfindungsgemäßen Verfahrens liegt das molare Verhältnis der Stoffmenge aller von der wässrigen Lösung **L₂** umfassten Salze **S** zur gesamten Stoffmenge aller in Schritt (a) des erfindungsgemäßen Verfahrens eingesetzten Verbindungen der Formeln **(III)** und **(IV)** im Bereich 10 : 1 bis 1 : 1000, bevorzugter im Bereich 1 : 1 zu 1 : 100, noch bevorzugter im Bereich 1 : 10 bis 1 : 50, am bevorzugtesten bei 1 : 21.

In einer alternativen bevorzugten Ausführungsform ergibt sich die Gesamtmenge der von der wässrigen Lösung **L₂** umfassten Salze **S** bezogen auf die Menge der in Schritt (a) des erfindungsgemäßen Verfahrens eingesetzten Verbindungen der Formel **(III)** bzw. **(IV)** wie folgt:
In dieser alternativen bevorzugten Ausführungsform umfasst die wässrige Lösung **L₂** pro Mol an in Schritt (a) des erfindungsgemäßen Verfahrens eingesetzter Verbindung der Formel **(III)** 0.001 bis 10 Mol, bevorzugt 0.01 bis 1 Mol, bevorzugter 0.025 bis 0.1 Mol, am bevorzugtesten 1/21 Mol an Salzen **S,** und zusätzlich pro Mol an in Schritt (a) des erfindungsgemäßen Verfahrens eingesetzter Verbindung der Formel **(IV)** p × [0.001 bis 10] Mol, bevorzugt p × [0.01 bis 1] Mol, bevorzugter p × [0.025 bis 0.1] Mol, am bevorzugtesten p × 1/21 Mol an Salzen **S.**

Es ist selbstverständlich dass, wenn im erfindungsgemäßen Verfahren mehrere Verbindungen der Formel **(IV),** welche sich durch den Wert von m bzw. p unterscheiden, eingesetzt werden, dass dann die bevorzugte Menge für jede Gruppe mit dem selben Wert für p wiederholt werden und dann die pro Gruppe berechneten Mengen an Salzen **S** addiert werden müssen.

Bei Verfahren zur Herstellung von Verbindungen der Formel **(I)** umfasst die wässrige Lösung **L₂** pro Mol an in Schritt (a) des erfindungsgemäßen Verfahrens eingesetzter Verbindung der Formel **(III)** insbesondere 0.001 bis 10 Mol, bevorzugt 0.01 bis 1 Mol, bevorzugter 0.025 bis 0.1 Mol, am bevorzugtesten 1/21 Mol an Salzen **S.**

Im erfindungsgemäßen Verfahren wird in Schritt (a) bevorzugt eine Verbindung der Formel **(III)** eingesetzt, so dass das erfindungsgemäße Verfahren eines zur Herstellung einer Verbindung der Formel **(I)** ist. Dabei sind noch bevorzugter R¹, R², R³, R⁴, R⁵, R⁶, R⁷ jeweils Methyl und R⁹, R¹⁰ jeweils Wasserstoff. Bei X^{a-} handelt es sich dann insbesondere um ein einwertiges organisches Anion, bevorzugt Methylsulfat oder ein Halogenid, welches noch bevorzugter aus der Gruppe bestehend aus Chlorid, Bromid, Jodid ausgewählt ist. Am bevorzugtesten ist X^{a-} dann Chlorid. Die Variablen a und n haben dann jeweils den Wert 1.

Im erfindungsgemäßen Verfahren wird H₂O₂ eingesetzt, insbesondere als wässrige Lösung mit einem Gehalt von 20 bis 50 Gew.-% (Gew.-% bedeutet Massenanteil), bevorzugt 30 bis 50 Gew.-% H₂O₂ in Wasser.

Im erfindungsgemäßen Verfahren liegt insbesondere das molare Verhältnis der Gesamtstoffmenge aller in Schritt (a) eingesetzten Verbindungen der Formeln **(III)** und **(IV)** zur Gesamtstoffmenge des in Schritt (a) eingesetzten H₂O₂ im Bereich 9 : 1 bis 1 : 9, bevorzugt 1 : 1.05 bis 1 : 9, bevorzugter 1 : 1.2 bis 1 : 5, noch bevorzugter 1 : 1.3 bis 1 : 3, noch bevorzugter 1 : 1.4 bis 1 : 2, am bevorzugtesten 1 : 1 bis 1 : 1.68.

In einer alternativen bevorzugten Ausführungsform wird die Menge des in Schritt (a) eingesetzten H₂O₂ bezogen auf die Menge der in Schritt (a) des erfindungsgemäßen Verfahrens eingesetzten Verbindungen der Formel **(III)** bzw. **(IV)** bestimmt.

In dieser alternativen bevorzugten Ausführungsform werden pro Mol an in Schritt (a) eingesetzter Verbindung der Formel **(III)** 1.1 bis 9 Mol H₂O₂, bevorzugt 1.25 bis 4 Mol H₂O₂, bevorzugter 1.4 bis 2.67 Mol H₂O₂, noch bevorzugter 1.5 bis 2.5 Mol H₂O₂, am bevorzugtesten 1.60 bis 1.68 Mol H₂O₂ in Schritt (a) eingesetzt und pro Mol an in Schritt (a) eingesetzter Verbindung der Formel **(IV)** p × [1.1 bis 9] Mol H₂O₂, bevorzugt p × [1.25 bis 4] Mol H₂O₂, bevorzugter p × [1.4 bis 2.67] Mol H₂O₂, noch bevorzugter p × [1.5 bis 2.5] Mol H₂O₂, am bevorzugtesten p × [1.60 bis 1.68] Mol H₂O₂ in Schritt (a) eingesetzt.

Es ist selbstverständlich dass, wenn im erfindungsgemäßen Verfahren mehrere Verbindungen der Formel **(IV),** welche sich durch den Wert von m bzw. p unterscheiden, eingesetzt werden, dass dann die bevorzugte Menge an H₂O₂ für jede Gruppe mit dem selben Wert für p wiederholt werden und dann die pro Gruppe berechneten Mengen an H₂O₂ addiert werden müssen.

Bei Verfahren zur Herstellung von Verbindungen der Formel **(I)** werden insbesondere pro Mol an in Schritt (a) eingesetzter Verbindung der Formel **(III)** 1.1 bis 9 Mol H₂O₂, bevorzugt 1.25 bis 4 Mol H₂O₂, bevorzugter 1.4 bis 2.67 Mol H₂O₂, noch bevorzugter 1.5 bis 2.5 Mol H₂O₂, am bevorzugtesten 1.60 bis 1.68 Mol H₂O₂ in Schritt (a) eingesetzt.

Das erfindungsgemäße Verfahren wird in der wässrigen Lösung **L₂** durchgeführt. Dies ist beim Einsatz von Wasserstoffperoxid als Peroxid schon allein dadurch der Fall, dass dabei stets Wasser mit in die Reaktion eingetragen werden. Der Anteil von Wasser in der wässrigen Lösung **L₂** liegt dabei insbesondere im Bereich 10 Gew.-% bis 90 Gew.-%, bevorzugt 20 Gew.-% bis 80 Gew.-%, bevorzugter 30 Gew.-% bis 70 Gew.-%, jeweils bezogen auf das Gesamtgewicht der wässrigen Lösung **L₂** (Gew.-% bedeutet Massenanteil).

In einer bevorzugten Ausführungsform des erfindungsgemäßen Verfahrens umfasst die wässrige Lösung **L₂,** gerade wenn das erfindungsgemäße Verfahren in Edelstahlreaktoren durchgeführt wird, Stabilisatoren für H₂O₂. Solche Stabilisatoren sind dem Fachmann bekannt und beispielsweise beschrieben in US 4,294,575, US 4,304,762, CA 1,152,292, US 4,034,064, US 4,061,721, US 4,362,706. Insbesondere werden Stabilisatoren ausgewählt aus der Gruppe bestehend aus Pyrophsophaten, bevorzugt Natriumpyrophosphaten, Phosphaten, Stannaten, Magnesiumionen, Silicationen, aminsubstituierten Organophosphonsäuren und deren Alkalisalzen. Es versteht sich von selbst, dass der Schritt (a) des erfindungsgemäßen Verfahrens möglichst vollständig ablaufen muss, bevor der Folgeschritt (b) bzw. (c) erfolgt.

Es ist deshalb bevorzugt, dass sich in Schritt (a) des erfindungsgemäßen Verfahrens mindestens 10 Mol.-%, bevorzugt mindestens 60 Mol.-%, bevorzugter mindestens 70 Mol.-%, noch bevorzugter mindestens 80 Mol.-%, noch mehr bevorzugter mindestens 90 Mol.-%, am bevorzugtesten mindestens 99 Mol.-% aller darin eingesetzten Verbindungen der Formeln **(III)** und **(IV)** zu den Verbindungen der Formel **(I)** und **(II)** umsetzen. Erst wenn diese Umsetzung erreicht ist, erfolgen in dieser bevorzugten Ausführungsform die Schritte (b) bzw. (c). Am bevorzugtesten ist die Umsetzung der in Schritt (a) des erfindungsgemäßen Verfahrens eingesetzten Verbindungen der Formeln **(III)** und **(IV)** zu den Verbindungen der Formel **(I)** und **(II)** vollständig, bevor zu Schritten (b) bzw. (c) übergegangen wird.

Nach Beendigung von Schritt (a) des erfindungsgemäßen Verfahrens umfasst die dann erhaltene Lösung gegebenenfalls H₂O₂, insbesondere wenn dieses in Schritt (a) im molaren Überschuss zur molaren Gesamtmenge der Verbindungen **(III)** und **(IV)** eingesetzt wurde.

Die vorliegende Erfindung zeichnet sich nun dadurch aus, dass die Aufarbeitung dieser Lösung gegenüber den im Stand der Technik wie WO 2018/028830 A1 beschriebenen Verfahren weniger aufwendig ist. So sind Fällung und Filtration eines Niederschlags nicht nötig. Der Grund dafür liegt im Einsatz der Hydrogencarbonatsalze bzw. Carbonatsalze, die eine leichte Aufarbeitung ermöglichen.

### 2. Schritt (b)

Schritt (b) des erfindungsgemäßen Verfahrens wird dann durchgeführt, falls die wässrige Lösung **L₂** nach der Umsetzung der mindestens einen Verbindung der Formel **(III)** oder **(IV)** mit H₂O₂ zur mindestens einen Verbindung der Formel **(I)** oder **(II)** noch H₂O₂ enthält.

Das ist insbesondere dann der Fall, wenn in Schritt (a) des erfindungsgemäßen Verfahrens H₂O₂ im molaren Überschuss bezogen auf die Gesamtmenge an Verbindungen **(III)** und **(IV)** eingesetzt wird.

In diesem Fall wird H₂O₂ aus der wässrigen Lösung **L₂** in Schritt (b) entfernt. "Entfernung" bedeutet in diesem Fall insbesondere, dass die nach Schritt (b) erhaltene wässrige Lösung **L₃** einen Gehalt an H₂O₂ aufweist, der < 10 Mol.-%, bevorzugt < 5 Mol.-%, bevorzugter < 1 Mol.-%, noch bevorzugter < 0.1 Mol.-% beträgt, bezogen auf den Gehalt an H₂O₂ in der wässrigen Lösung **L₂** nach Durchführung des Schrittes (a) des erfindungsgemäßen Verfahrens.

Die Entfernung von H₂O₂ im Schritt (b) des erfindungsgemäßen Verfahrens erfolgt insbesondere über zwei bevorzugte alternative Verfahren: 2.1 In einer ersten bevorzugten Ausführungsform des Schritts (b) wird nach der Umsetzung gemäß Schritt (a) H₂O₂ durch Einstellen der wässrigen Lösung **L₂** auf einen pH-Wert von ≥ 10.5 entfernt. Noch bevorzugter wird ein pH-Wert im Bereich von 10.5 bis 12, noch mehr bevorzugter 10.6 bis 11.5, am bevorzugtesten ein pH von 11 eingestellt.

Die Einstellung des pH-Wertes der wässrigen Lösung **L₂** in Schritt (b) erfolgt dabei insbesondere durch Zugabe einer Base, insbesondere ausgewählt aus der Gruppe bestehend aus Alkalimetallhydroxid, Erdalkalimetallhydroxid. Als Alkalimetallhydroxid, Erdalkalimetallhydroxid wird praktischerweise jenes verwendet, dessen Kation mit dem Kation mindestens eines der in Schritt (a) eingesetzten Salze **S** übereinstimmt.

Am bevorzugtesten wird dabei NaOH als Base eingesetzt.

2.2 In einer zweiten bevorzugten Ausführungsform des Schritts (b) wird nach der Umsetzung gemäß Schritt (a) H₂O₂ durch Umsetzen der wässrigen Lösung **L₂** in Gegenwart eines Katalysators entfernt. Der Katalysator ist dabei bevorzugt aus der Gruppe bestehend aus Trägermetallkatalysatoren, Metallvollkatalysatoren, Metalloxide, Enzyme ausgewählt.

2.2.1 Trägermetallkatalysatoren sind dem Fachmann bekannt und insbesondere in der EP 0 302 020 A2 beschrieben.

Trägermetallkatalysatoren umfassen mindestens ein, bevorzugt elementar vorliegendes, Metall **M_{T}**.

Das Metall **M_{T}** ist dabei insbesondere ausgewählt aus der Gruppe bestehend aus V, Cr, Mo, Mn, Ni, Pd, Pt, Fe, Ru, Os, Co, Rh, Ir, Cu; bevorzugt ausgewählt aus der Gruppe bestehend aus V, Cr, Mo, Mn, Ni, Pd, Pt, Fe, Ru, Os, Co, Rh, Ir, Cu; bevorzugter ausgewählt aus der Gruppe bestehend aus Cr, Mo, Mn, Ni, Pd, Pt, Ru, Rh; noch bevorzugter ausgewählt aus der Gruppe bestehend aus Cr, Ni, Pd, Pt; am bevorzugtesten ausgewählt aus der Gruppe bestehend aus Pt, Pd.

Bei einem Trägermetallkatalysator ist das Metall **M_{T}** auf einem dem Fachmann bekannten Träger aufgebracht, welcher insbesondere ausgewählt aus der Gruppe bestehend aus Aktivkohle, Calciumcarbonat, Aluminiumoxid, Titandioxid, bevorzugt aus der Gruppe bestehend aus Aluminiumoxid, Aktivkohle ausgewählt ist. Am bevorzugtesten ist als Träger Aluminiumoxid, Al₂O₃.

Der Anteil an Metall **M_{T}** im Trägermetallkatalysator ist dabei nicht besonders beschränkt und liegt insbesondere im Bereich 0.1 bis 30 Gew.-%, bevorzugt 1 bis 10 Gew.-%, bevorzugter 5 Gew.-%. Dabei bedeutet Gew.-% das Gesamtgewicht aller vom jeweiligen Trägermetallkatalysator umfassten Metalle **M_{T}** bezogen auf das Gesamtgewicht des vom jeweiligen Trägermetallkatalysator umfassten Trägers.

Wird in Schritt (b) des erfindungsgemäßen Verfahrens H₂O₂ aus **L₂** mit einem Trägermetallkatalysator entfernt, wird der Trägermetallkatalysator zur wässrigen Lösung **L₂** zugegeben und H₂O₂ an diesem Trägerkatalysator zu H₂O und O₂ disproportioniert.

Dabei wird die Temperatur in Schritt (b) bevorzugt bei 20 °C bis 80 °C, bevorzugter bei 40 °C bis 70 °C, noch bevorzugter bei 50 °C bis 60 °C, am bevorzugtesten bei 60 °C eingestellt.

Insbesondere beträgt das Gewicht (Massenanteil) des zugegebenen Trägerkatalysators 0.1 % bis 5 %, bevorzugter 0.5 % bis 2 %, bezogen auf das Gesamtgewicht der wässrigen Lösung **L₂**. Bevorzugt, insbesondere bei kontinuierlicher Fahrweise des erfindungsgemäßen Verfahrens, wird bei Umsetzen der wässrigen Lösung **L₂** in Gegenwart eines Trägermetallkatalysators in Schritt (b) die wässrige Lösung **L₂** über einem Katalysatorbett umfassend den entsprechenden Trägermetallkatalysator umgesetzt. Diese bevorzugte Ausführungsform hat den zusätzlichen Vorteil, dass ein Filtrationsschritt, in dem der Trägermetallkatalysator entfernt werden muss, entfällt.

2.2.2 Metallvollkatalysatoren sind dem Fachmann bekannt und beispielsweise in EP 3 266 766 A1 beschrieben. Ein "Metallvollkatalysator" ist ein vollständig von dem katalytischen Material durchdrungener Formkörper. Er unterscheidet sich damit vom "Trägermetallkatalysator", bei dem die katalytisch aktive Komponente auf einem von der katalytisch aktiven Komponente verschiedenen Träger aufgebracht ist.

Metallvollkatalysatoren weisen erfindungsgemäß mindestens ein elementares Metall **M_{V}** auf, wobei das elementare Metall **M_{V}** bevorzugt ausgewählt ist aus der Gruppe bestehend aus Ag, V, Cr, Mo, Mn, Ni, Pd, Pt, Fe, Ru, Os, Co, Rh, Ir, Cu; insbesondere ausgewählt ist aus der Gruppe bestehend aus Ag, Fe, Cr, Mo, Mn, Ni, Co, Cu, Pd, Pt, Ru, Rh; bevorzugt ausgewählt ist aus der Gruppe bestehend aus Ag, Fe, Cr, Ni, Co, Cu, Pd, Pt; bevorzugter ausgewählt ist aus der Gruppe bestehend aus Ag, Fe, Ni, Co, Cu, Pd, Pt; noch bevorzugter ausgewählt ist aus der Gruppe bestehend aus Ag, Fe, Ni, Co, Cu; noch mehr bevorzugter ausgewählt ist aus der Gruppe bestehend Co, Ni; am bevorzugtesten Ni ist.

Der Metallvollkatalysator kann eine Legierung des Metalls **M_{V}** sein (wobei das Metall **M_{V}** zu mindestens > 50 Gew.-% in der Legierung vorliegt, bezogen auf das Gesamtgewicht der Legierung) und beispielsweise außer **M_{V}** noch mindestens ein Metall bzw. Halbmetall ausgewählt aus Al, Si, Mg, Zn, Mo, Cr, insbesondere Al, aufweisen.

Noch mehr bevorzugter weist der Metallvollkatalysator mindestens ein Metall **M_{V}** ausgewählt aus der Gruppe bestehend aus Raney-Kobalt, Raney-Kupfer, Raney-Silber, Raney-Eisen, Raney-Nickel, insbesondere ausgewählt aus Raney-Nickel, Raney-Kobalt, am bevorzugtesten ausgewählt aus Raney-Nickel auf.

Die Synthese dieser Metallvollkatalysatoren sind dem Fachmann bekannt und beispielsweise in US 1,629,190, DE 20 2010 007837 U1 beschrieben. Dazu legiert man Ni mit Al, Si, Mg oder Zn (insbesondere mit Al, bevorzugt im Verhältnis 1:1) und löst aus der Legierung nach mechanischer Zerkleinerung mit Alkalien (insbesondere NaOH) das katalytisch unwirksame Metall (Al) mindestens teilweise heraus.

Entsprechend werden auch Raney-Kupfer, Raney-Kobalt, Raney Silber oder Raney-Eisen hergestellt (beschrieben zum Beispiel in der DE 20 2010 007837 U1).

Wird in Schritt (b) des erfindungsgemäßen Verfahrens H₂O₂ aus **L₂** mit einem Metallvollkatalysator entfernt, wird der Metallvollkatalysator zur wässrigen Lösung **L₂** zugegeben und H₂O₂ an diesem Metallvollkatalysator zu H₂O und O₂ disproportioniert.

Dabei wird die Temperatur in Schritt (b) bevorzugt bei 20 °C bis 80 °C, bevorzugter bei 40 °C bis 70 °C, noch bevorzugter bei 50 °C bis 60 °C, am bevorzugtesten bei 60 °C eingestellt.

Insbesondere beträgt das Gewicht (Massenanteil) des zugegebenen Vollkatalysators 0.1 % bis 5 %, bevorzugter 0.5 % bis 2 %, bezogen auf das Gesamtgewicht der wässrigen Lösung **L₂**. Bevorzugt, insbesondere bei kontinuierlicher Fahrweise des erfindungsgemäßen Verfahrens, wird bei Umsetzen der wässrigen Lösung **L₂** in Gegenwart eines Metallvollkatalysators in Schritt (b) die wässrige Lösung **L₂** über einem Katalysatorbett umfassend den entsprechenden Metallvollkatalysator umgesetzt. Diese bevorzugte Ausführungsform hat den zusätzlichen Vorteil, dass ein Filtrationsschritt, in dem der Metallvollkatalysator entfernt werden muss, entfällt.

2.2.3 Wird in Schritt (b) des erfindungsgemäßen Verfahrens H₂O₂ mit einem Metalloxidkatalysator entfernt, wird der Metalloxidkatalysator zur wässrigen Lösung **L₂** zugegeben und H₂O₂ an diesem Metallvollkatalysator zu H₂O und O₂ disproportioniert.

Dabei wird die Temperatur in Schritt (b) bevorzugt bei 20 °C bis 80 °C, bevorzugter bei 40 °C bis 70 °C, noch bevorzugter bei 50 °C bis 60 °C, am bevorzugtesten bei 60 °C eingestellt.

Insbesondere beträgt das Gewicht (Massenanteil) des zugegebenen Metalloxidkatalysators 0.1 % bis 5 %, bevorzugter 0.5 % bis 2 %, bezogen auf das Gesamtgewicht der wässrigen Lösung **L₂**. Bevorzugt, insbesondere bei kontinuierlicher Fahrweise des erfindungsgemäßen Verfahrens, wird bei Umsetzen der wässrigen Lösung **L₂** in Gegenwart eines Metalloxidkatalysators in Schritt (b) die wässrige Lösung **L₂** über einem Katalysatorbett umfassend den entsprechenden Metalloxidkatalysator umgesetzt. Diese bevorzugte Ausführungsform hat den zusätzlichen Vorteil, dass ein Filtrationsschritt, in dem der Metalloxidkatalysator entfernt werden muss, entfällt.

Der bevorzugteste Metalloxidkatalysator ist Braunstein, MnO₂.

2.2.4 Wird in Schritt (b) des erfindungsgemäßen Verfahrens H₂O₂ durch enzymatische Katalyse entfernt, wird das jeweilige Enzym zur wässrigen Lösung **L₂** zugegeben und H₂O₂ durch dieses zu H₂O und O₂ disproportioniert.

Als Enzym wird insbesondere bovine Katalase, wie beispielsweise von M. Ibrahim, H. G. Schlegel, Biotechnol Bioeng. 1980, 22, 1895-1906 beschrieben, genutzt.

Bevorzugt wird der Schritt (b) wie unter Punkt 2.1 beschrieben durchgeführt. Dies hat den Vorteil, dass keine Filtration der wässrigen Lösung **L₂** durchgeführt werden muss, sondern diese direkt dem Folgeschritt (c) zugeführt werden kann.

Von den Punkten 2.2.1 bis 2.2.3, sind die Verfahren wie unter Punkten 2.2.1 bis 2.2.2 beschrieben bevorzugter, noch bevorzugter das unter Punkt 2.2.1 beschriebene Verfahren.

Nach Durchführung von Schritt (a) bzw., wenn Schritt (b) durchgeführt wird, von Schritt (b) wird eine wässrige Lösung **L₃** umfassend mindestens eine Verbindung der Formel **(I)** bzw. **(II)** und mindestens ein Salz **S** erhalten.

### 3. Schritt (c)

In Schritt (c) des erfindungsgemäßen Verfahrens wird ein saurer pH-Wert in **L₃** eingestellt.

In einer noch bevorzugteren Ausführungsform wird dabei in **L₃** ein pH < 6.9, bevorzugt ein pH im Bereich von 1 bis 6, bevorzugter 2 bis 5, noch bevorzugter ein pH von 5 eingestellt.

Durch Einstellung des sauren pH-Wertes der wässrigen Lösung **L₃** wird das Salz **S** (der Katalysator, der für die Umsetzung in Schritt (a) nötig war), mindestens teilweise aus der wässrigen Lösung **L₃** entfernt, und es wird eine wässrige Elektrolytlösung **L₁** erhalten.

In einer bevorzugten Ausführungsform wird in Schritt (c) der Anteil an Hydrogencarbonatanionen und Carbonatanionen so verringert, dass die Summe der molaren Menge an Hydrogencarbonatanion und Carbonatanion in der wässrigen Lösung **L₁** < 10 Mol.-%, bevorzugt < 5 Mol.-%, bevorzugter < 1 Mol.-%, noch bevorzugter < 0.1 Mol.-% ist als der in der wässrigen Lösung **L₂**.

Der saure pH-Wert in **L₃** wird praktischerweise eingestellt, in dem der wässrigen Lösung eine anorganische oder organische, bevorzugt eine anorganische Säure zugegeben wird. Als anorganische Säuren eignen sich insbesondere Salzsäure, Phosphorsäure, Schwefelsäure, Trifluormethansulfonsäure, Salpetersäure, Perchlorsäure, Borsäure, Blausäure, Flusssäure, am bevorzugtesten Salzsäure.

Es wird bevorzugt, dass in der wässrigen Elektrolytlösung **L₁** der Anteil aller Verbindungen der Formeln **(I)** und **(II)** bzw. wenn nur Verbindungen der Formeln **(III)** als Edukte eingesetzt werden, der Formel **(I),** in der wässrige Lösung **L₁** im Bereich 10 bis 80 Gew.-%, bevorzugt 30 bis 70 Gew.-%, bezogen auf das Gesamtgewicht der wässrigen Elektrolytlösung **L₁** liegt.

Die im erfindungsgemäßen Verfahren erhaltene wässrige Elektrolytlösung **L₁** kann in Ladungsspeichereinheiten, insbesondere *Redox*-*flow*-Batterien, eingesetzt werden.

Das erfindungsgemäße Verfahren stellt insbesondere gegenüber jenen des Standes der Technik ein überraschend einfaches Verfahren zur Herstellung von wässrigen Elektrolytlösungen von mindestens einer Verbindung der Formeln **(I)** und **(II),** bevorzugt mindestens einer Verbindung der Formeln **(I)** dar. Sie zeichnet sich durch eine unkomplizierte Aufarbeitung der Reaktionslösungen auf und insbesondere dadurch, dass auf einen Filtrationsschritt verzichtet werden kann.

### 4. Edukte

Die im erfindungsgemäßen Verfahren eingesetzten Verbindungen der Formeln **(III)** und **(IV)** sind 2,2,6,6-Tetraalkylpiperidin-4-ammoniumsalze, deren Herstellung dem Fachmann geläufig sind. Deren Synthese ist beispielsweise in WO 2018/028830 A1 beschrieben.

In einer bevorzugten Ausführungsform des erfindungsgemäßen Verfahrens werden die eingesetzten Edukte der Formeln **(III)** und **(IV)** in einem vorhergehenden Schritt aus der Formel **(V)** erhalten mit:

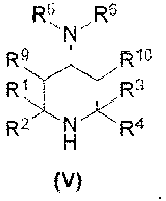

In der Formel **(V)** haben die Reste R¹, R², R³, R⁴, R⁵, R⁶, R⁹, R¹⁰ die vorgenannten Bedeutungen. Dazu wird die Verbindung mit der Formel **(V)** mit einem organischen Halogenid der Formel R⁷-Hal oder der Formel Hal-R⁸-(Hal)ₘ oder einem organischen Sulfat der Formel R⁷-O-SO₂-O-R⁷, worin R⁷, R⁸, m die vorgenannten Definitionen aufweisen, umgesetzt. Die Umsetzung mit dem genannten organischen Halogenid R⁷-Hal ist dabei bevorzugt. Hal ist dabei aus der Gruppe bestehend aus F, Cl, Br, I ausgewählt, bevorzugt aus der Gruppe bestehend aus Cl, Br, I und am bevorzugtesten Cl.

Diese Umsetzung läuft in einem Lösungsmittel ab. Dabei handelt es sich dabei bevorzugt um ein organisches Lösungsmittel, welches aus der Gruppe bestehend aus Ether, Nitril, halogenierter Kohlenwasserstoff, aromatischer Kohlenwasserstoff, aliphatischer Kohlenwasserstoff ausgewählt ist.

Bevorzugte Ether sind Diethylether und Di-isopropylether. Beispiele für Nitrile sind Acetonitril.

Bevorzugte halogenierte Kohlenwasserstoffe sind chlorierte und/oder fluorierte aliphatische Kohlenwasserstoffe, wie Dichlormethan, Chloroform, Tetrachlorkohlenstoff, Dichlorethan oder Chlorfluorethan.

Bevorzugte aromatische Kohlenwasserstoffe sind Benzol, Toluol oder Xylol. Beispiele für aliphatische Kohlenwasserstoffe sind Hexan, Octan oder Decan sowie Petrolether oder Petroleum.

Als R⁷-Hal werden bevorzugt Alkylchloride, Alkylbromide oder Alkyljodide eingesetzt, besonders bevorzugt aber Alkylchloride. Bevorzugte Beispiele für Alkylchloride der Formel R⁷-Cl sind Methylchlorid, Ethylchlorid. Ganz besonders bevorzugt ist Methylchlorid.

Beispiele dafür sind Alkylchloride, deren Alkylgruppe ein bis sechs Kohlenstoffatome aufweist. Bevorzugt befindet sich das Chloratom am endständigen Kohlenstoffatom.

Als Hal-R⁸-(Hal)ₘ eingesetzte Chloride weisen zwei bis sechs Chloratome auf, bevorzugt werden Alkyldi-, Alkyltri-, Alkylquater-, Alkylpenta- und Alkylhexachloride. Beispiele dafür sind Alkyldichloride oder Alkyltrichloride, deren Alkylengruppen oder Alkyltriylgruppen ein bis sechs Kohlenstoffatome aufweisen. Am bevorzugtesten ist 1,6-Dichlorhexan, 1,4-Dichlorbutan und Ethylendichlorid (EDC).

Bei R⁷-O-SO₂-O-R⁷ handelt es sich um ein organisches Sulfat. Bevorzugt werden Dialkylsulfate, insbesondere Dimethylsulfat.

Das molare Verhältnis von 4-Amino-alkylpiperidin der Formel **(V)** zu Alkylchlorid der Formel R⁷-Hal in der Umsetzung beträgt üblicherweise 1 : 5 bis 1 : 1 , insbesondere 1 : 3 bis 1 : 1 und ganz besonders bevorzugt 1 : 1.05 bis 1 : 1.

Das molare Verhältnis von 4-Amino-alkylpiperidin der Formel **(V)** zu organischem Chlorid der Formel Hal-R⁸-(Hal)ₘ beträgt üblicherweise 1 : 5 × m bis 1 : m, insbesondere 1 : 3 × m bis 1 : m und ganz besonders bevorzugt 1 : 2 × m bis 1 : m, worin m die oben definierte Bedeutung besitzt.

Das molare Verhältnis von 4-Amino-alkylpiperidin der Formel **(V)** zu organischem Sulfat der Formel R⁷-O-SO₂-O-R⁷ beträgt üblicherweise 1 : 5 bis 1 : 1 , insbesondere 1 : 3 bis 1 : 1 und ganz besonders bevorzugt 1 : 2 bis 1 : 1.

Die Umsetzung von Verbindungen der Formel **(V)** zu Verbindungen der Formel **(III)** oder **(IV)** erfolgt insbesondere bei Temperaturen im Bereich von 0 °C bis 200 °C, vorzugsweise von 25 °C bis 150 °C, und ganz besonders bevorzugt bei 40 °C bis 100 °C. Bevorzugte Drucke liegen dabei im Bereich von 1 bar bis 250 bar, vorzugsweise von 1 bar bis 100 bar, und ganz besonders bevorzugt bei 1 bar bis 6 bar. Die Umsetzung kann in An- und Abwesenheit einer Base durchgeführt werden. Insbesondere bei 4-Amino-alkylpiperidinylverbindungen der Formel **(V),** in denen die 4-Aminogruppe ein oder mehrere Wasserstoffatome aufweist, ist es bevorzugt, wenn die Umsetzung in Anwesenheit einer Base durchgeführt wird. Beispiele für geeignete Basen sind anionische lonenaustauscher, Alkalihydroxide, wie Natriumhydroxid oder Kaliumhydroxid, oder organische Amine, vorzugsweise solche Amine, die bei den Drucken und Temperaturen in der Umsetzung als flüssige Phase vorliegen.

Die Verbindungen der Formel **(V)** wiederum können nach dem Fachmann bekannten Verfahren, wie sie beispielsweise in der WO 2018/028830 A1 beschrieben sind, aus den entsprechenden 4-Oxo-Alkylpiperidinverbindungen oder den entsprechenden Iminen hergestellt werden.

### Allgemeine Begriffe

R⁸ ist eine p-wertige organische Brückengruppe, wobei p eine ganze Zahle zwischen zwei und sechs ist. Darunter ist demnach ein organischer Rest zu verstehen, der über zwei, drei, vier, fünf oder sechs kovalente Bindungen mit dem Rest des Moleküls verbunden ist. R⁸ ist bevorzugt Alkylen.

Beispiele für zweiwertige organische Brückengruppen sind Alkylen, Alkylenoxy, Poly(alkylenoxy), Alkylenamino, Poly(alkylenamino), Cycloalkylen, Arylen, Aralkylen oder Heterocyclylen.

Alkylengruppen können sowohl verzweigt als auch unverzweigt sein. Eine Alkylengruppe enthält typischerweise ein bis zu zwanzig Kohlenstoffatome, bevorzugt zwei bis zu sechs Kohlenstoffatome. Beispiele für Alkylengruppen sind: Methylen, Ethylen, Propylen und Butylen, Pentylen, Hexylen. Alkylengruppen können gegebenenfalls substituiert sein, beispielsweise mit Alkyl, Alkoxy, Hydroxy, Carboxyl, Carboxylester, Carboxylamid, Sulfonat, Sulfonsäureester, Sulfonamid, Halogen.

Alkylenoxy- und Poly(alkylenoxy)gruppen können sowohl verzweigte als auch unverzweigte Alkylengruppen enthalten. Eine in einer Alkylenoxy- oder in einer Poly(alkylenoxy)gruppe auftretende Alkylengruppe enthält typischerweise zwei bis vier Kohlenstoffatome, bevorzugt zwei oder drei Kohlenstoffatome. Die Anzahl der Wiederholeinheiten in den Poly(alkylenoxy)gruppen kann in weiten Bereichen schwanken. Typische Anzahlen von Wiederholeinheiten bewegen sich im Bereich von 2 bis 50. Beispiele für Alkylenoxygruppen sind: Ethylenoxy, Propylenoxy und Butylenoxy. Beispiele für Poly(alkylenoxy)gruppen sind: Poly(ethylenoxy), Poly(propylenoxy) und Poly(butylenoxy).

Alkylenamino- und Poly(alkylenamino)gruppen können sowohl verzweigte als auch unverzweigte Alkylengruppen enthalten. Eine in einer Alkylenamino- oder in einer Poly(alkylenamino)gruppe auftretende Alkylengruppe enthält typischerweise zwei bis vier Kohlenstoffatome, bevorzugt zwei oder drei Kohlenstoffatome. Die Anzahl der Wiederholeinheiten in den Poly(alkylenamino)gruppen kann in weiten Bereichen schwanken. Typische Anzahlen von Wiederholeinheiten bewegen sich im Bereich von 2 bis 50. Beispiele für Alkylenaminogruppen sind: Ethylenamino, Propylenamino und Butylenamino. Beispiele für Poly(alkylenamino)gruppen sind: Poly(ethylenamino), Poly(propylenamino) und Poly(butylenamino).

Cycloalkylengruppen enthalten typischerweise fünf, sechs oder sieben Ringkohlenstoffatome, die jeweils unabhängig voneinander substituiert sein können. Beispiele für Substituenten sind Alkylgruppen oder zwei Alkylgruppen, die zusammen mit den Ringkohlenstoffen, an denen sie gebunden sind, einen weiteren Ring bilden können. Ein Beispiel für eine Cycloalkylengruppe ist Cyclohexylen.

Arylengruppen sind typischerweise cyclische aromatische Gruppen, die fünf bis vierzehn Kohlenstoffatome enthalten, die jeweils unabhängig voneinander substituiert sein können. Beispiele für Arylengruppen sind o-Phenylen, m-Phenylen, p-Phenyl, Naphthylengruppen oder Biphenylengruppen.

Heterocyclylgruppen sind typischerweise cyclische Gruppen mit vier bis zehn Ringkohlenstoffatomen und mindestens einem Ringheteroatom, die jeweils unabhängig voneinander substituiert sein können, insbesondere mit Alkyl. Beispiele für Heteroatome sind Sauerstoff, Stickstoff, Phosphor, Bor, Selen oder Schwefel. Beispiele für Heterocyclylengruppen sind Furandiyl, Thiophendiyl, Pyrroldiyl oder Imidazoldiyl. Heterocyclylengruppen sind vorzugsweise aromatisch.

Aralkylengruppen sind typischerweise Arylgruppen, an die ein oder zwei Alkylgruppen kovalent gebunden sind. Aralkylgruppen können über ihren Arylrest und ihren Alkylrest oder über zwei Alkylreste mit dem Rest des Moleküls kovalent verbunden sein. Die Aralkylengruppe kann am aromatischen Ring beispielsweise mit Alkylgruppen oder mit Halogenatomen substituiert sein. Beispiele für Aralkylengruppen sind Benzylen oder Dimethylphenylen (Xylylen).

Beispiele für dreiwertige organische Reste R⁸ sind Alkyltriyl, Alkoxytriyl, Trispoly(alkylenoxy), Tris-poly(alkylenamino), Cycloalkyltriyl, Aryltriyl, Aralkyltriyl oder Heterocyclyltriyl. Diese Reste entsprechen den bereits weiter oben eingehend beschriebenen zweiwertigen Resten mit dem Unterschied, dass diese mit drei kovalenten Bindungen anstelle von zwei kovalenten Bindungen mit dem Rest des Moleküls verbunden sind.

Beispiele für vierwertige organische Reste R⁸ sind Alkylquaternyl, Alkoxyquaternyl, Quater-poly(alkylenoxy), Quaterpoly(alkylenamino), Cycloalkylquaternyl, Arylquaternyl, Aralkylquaternyl oder Heterocyclylquaternyl. Diese Reste entsprechen den bereits weiter oben eingehend beschriebenen zweiwertigen Resten mit dem Unterschied, dass diese mit vier kovalenten Bindungen anstelle von zwei kovalenten Bindungen mit dem Rest des Moleküls verbunden sind.

Beispiele für fünfwertige organische Reste R⁸ sind Alkylquinquinyl, Alkoxyquinquinyl, Quinqui-poly(alkylenoxy), Quinquipoly(alkylenamino), Cycloalkylquinquinyl, Arylquinquinyl, Aralkylquinquinyl oder Heterocyclylquinquinyl. Diese Reste entsprechen den bereits weiter oben eingehend beschriebenen zweiwertigen Resten mit dem Unterschied, dass diese mit fünf kovalenten Bindungen anstelle von zwei kovalenten Bindungen mit dem Rest des Moleküls verbunden sind.

Beispiele für sechswertige organische Reste R⁸ sind Alkylhexyl, Alkoxyhexyl, Hexyl-poly(alkylenoxy), Hexylpoly(alkylenamino), Cycloalkylhexyl, Arylhexyl, Aralkylhexyl oder Heterocyclylhexyl. Diese Reste entsprechen den bereits weiter oben eingehend beschriebenen zweiwertigen Resten mit dem Unterschied, dass diese mit sechs kovalenten Bindungen anstelle von zwei kovalenten Bindungen mit dem Rest des Moleküls verbunden sind.

Beispiele für anorganische Anionen X^{a-} sind Halogenidionen, wie Fluorid, Chlorid, Bromid oder Jodid, oder Hydroxidionen oder Anionen anorganischer Säuren, wie Phosphat, Sulfat, Nitrat, Borat, Hexafluorophosphat, Tetrafluoroborat, Perchlorat, Chlorat, Hexafluoroantimonat, Hexafluoroarsenat, Cyanid. Zu den Sulfat- oder Phospatanionen zählen auch solche, die einen organischen Rest aufweisen, insbesondere einen Alkylrest, ganz besonders bevorzugt einen Methylrest. Bevorzugt wird das Methylsulfatanion CH₃-OSO₃⁻.

Beispiele für organische Anionen X^{a-} sind Anionen ein- oder mehrwertiger Carbonsäuren oder ein- oder mehrwertiger Sulfonsäuren, wobei diese Säuren gesättigt oder ungesättigt sein können. Weitere Beispiele für organische Anionen X^{a-} sind Anionen von Polycarbonsäuren oder von Polysulfonsäuren. Beispiele für Anionen organischer Säuren sind Acetat, Formiat, Trifluoroacetat, Trifluormethansulfonat, Pentafluorethansulfonat, Nonofluorbutansulfonat, Butyrat, Citrat, Fumarat, Glutarat, Lactat, Malat, Malonat, Oxalat, Pyruvat oder Tartrat. Beispiele für Anionen von Polycarbonsäuren sind Polyacrylat- oder Polymethacrylatanionen. Ein Beispiel für Anionen von Polysulfonsäuren ist Polystyrensulfonat (PSS).

Bedeutet einer der Reste R⁵, R⁶, R⁵', R⁶' und/oder R⁷ Alkyl, so kann die Alkylgruppe sowohl verzweigt als auch unverzweigt sein. Eine Alkylgruppe dieser Reste enthält typischerweise ein bis zu zwanzig Kohlenstoffatome, bevorzugt ein bis zu zehn Kohlenstoffatome. Beispiele für Alkylgruppen sind: Methyl, Ethyl, *n*-Propyl, *iso*-Propyl, *n*-Butyl, *sec*-Butyl, *tert*-Butyl, Pentyl, *n*-Hexyl, *n*-Heptyl, 2-Ethylhexyl, *n*-Octyl, *n*-Nonyl, *n*-Decyl, *n*-Undecyl, *n*-Dodecyl, *n*-Tridecyl, *n*-Tetradecyl, *n*-Pentadecyl, *n*- Hexadecyl, *n*-Heptadecyl, *n*-Octadecyl, *n*-Nonadecyl oder Eicosyl. Besonders bevorzugt sind von den vorgenannten Alkylgruppen jene mit ein bis sechs, noch bevorzugter jene mit ein bis vier Kohlenstoffatomen.

Die Reste R⁵, R⁶, R⁵', R⁶' und/oder R⁷ als Alkyl können gegebenenfalls substituiert sein, beispielsweise mit Alkoxy, Hydroxy, Carboxyl, Carboxylester, Carboxylamid, Sulfonat, Sulfonsäureester, Sulfonamid oder Halogen, bevorzugt mit Alkoxy, Hydroxy, Halogen. Der Rest R⁷ als Alkyl kann gegebenenfalls auch mit Nitro substituiert sein.

Die Reste R⁵, R⁶, R⁵', R⁶' und/oder R⁷ als Alkyl können gegebenenfalls durch ein oder mehrere, nicht direkt zueinander benachbarte zweiwertige Sauerstoff- oder Iminoreste unterbrochen sein. Beispiele dafür sind einwertige Reste abgeleitet von Polyethylenglykolen, von Polypropylenglykolen, von Polyethyleniminen oder von Polypropyleniminen.

Bedeutet einer der Reste R⁵, R⁶, R⁵' und/oder R⁶' Cycloalkyl, so ist die Cycloalkylgruppe typischerweise eine cyclische Gruppe, enthaltend fünf bis acht, vorzugsweise fünf, sechs oder sieben Ringkohlenstoffatome, die jeweils unabhängig voneinander substituiert sein können. Beispiele für Substituenten sind Alkylgruppen oder zwei Alkylgruppen, die zusammen mit den Ringkohlenstoffen, an denen sie gebunden sind, einen weiteren Ring bilden können. Beispiele für Cycloalkylgruppen sind Cyclopentyl oder Cyclohexyl. Cycloalkylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵, R⁶, R⁵' und/oder R⁶' Aryl, so ist die Arylgruppe typischerweise eine cyclische aromatische Gruppe, enthaltend fünf bis vierzehn Kohlenstoffatome, die jeweils unabhängig voneinander substituiert sein können. Beispiele für Substituenten sind Alkylgruppen oder zwei Alkylgruppen, die gemeinsam mit den Ringkohlenstoffatomen, an denen sie gebunden sind, einen weiteren Ring bilden können. Beispiele für Arylgruppen sind Phenyl, Biphenyl, Anthryl oder Phenantolyl. Arylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵, R⁶, R⁵' und/oder R⁶' Heterocyclyl, so kann die Heterocyclylgruppe typischerweise eine cyclische Gruppe mit vier bis zehn Ringkohlenstoffatomen und mindestens einem Ringheteroatom aufweisen, die jeweils unabhängig voneinander substituiert sein können.

Beispiele für Substituenten der Heterocyclylgruppe sind Alkylgruppen oder zwei Alkylgruppen die zusammen mit den Ringkohlenstoffen an denen sie gebunden sind einen weiteren Ring bilden können. Beispiele für Heteroatome sind Sauerstoff, Stickstoff, Phosphor, Bor, Selen oder Schwefel. Beispiele für Heterocyclyigruppen sind Furyl, Thienyl, Pyrrolyl oder Imidazolyl. Heterocyclyigruppen sind vorzugsweise aromatisch. Heterocyclyigruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵, R⁶, R⁵' und/oder R⁶' Aralkyl, so ist die Aralkylgruppe typischerweise eine Arylgruppe, wobei Aryl zuvor bereits definiert wurde, an die kovalent eine Alkylgruppe gebunden ist. Die Aralkylgruppe kann am aromatischen Ring beispielsweise mit Alkylgruppen oder mit Halogenatomen substituiert sein. Ein Beispiel für eine Aralkylgruppe ist Benzyl. Aralkylgruppen können gegebenenfalls mit Alkoxy, Hydroxy, Carboxyl, Carboxylester, Carboxylamid, Sulfonat, Sulfonsäureester, Sulfonamid oder Halogen, bevorzugt mit Alkoxy, Hydroxy, Halogen substituiert sein.

In einer bevorzugten Ausführungsform der vorliegenden Erfindung sind R⁵, R⁶, R⁵', R⁶' unabhängig voneinander jeweils Alkyl, welches gegebenenfalls mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid oder Halogen substituiert sind und/oder die gegebenenfalls in der Alkylgruppe durch ein oder mehrere, nicht direkt zueinander benachbarte zweiwertige Reste ausgewählt aus der Gruppe bestehend aus Sauerstoff, Imino unterbrochen sind.

In einer weiteren bevorzugten Ausführungsform der vorliegenden Erfindung ist R⁷ Alkyl, das gegebenenfalls mit ein oder mehreren Resten ausgewählt aus der Gruppe bestehend aus Alkoxy, Hydroxy, Nitro, Carboxyl, Carboxylester, Carboxylamid, Sulfonat, Sulfonsäureester, Sulfonamid oder Halogen substituiert ist und/oder das gegebenenfalls in der Alkylgruppe durch ein oder mehrere, nicht direkt zueinander benachbarte zweiwertige Reste ausgewählt aus der Gruppe bestehend aus Sauerstoff, Imino unterbrochen ist.

Ganz besonders bevorzugt bedeuten R⁵, R⁶, R⁵', R⁶' und/oder R⁷ unabhängig voneinander C₁-C₆-Alkyl.

Bedeutet einer der Substituenten Halogen, so ist darunter ein kovalent gebundenes Fluor-, Chlor-, Brom- oder Jodatom zu verstehen. Bevorzugt ist als Halogen Chlor oder Brom.

### Beispiel

### Erfinderisches Beispiel

Ein Reaktor wurde mit 950 kg entionisiertem Wasser beladen und auf 60 °C erhitzt. Unter Rühren wurden 28 kg (333 Mol) Natriumhydrogencarbonat zugegeben. Unter Rühren wurden schließlich 1644 kg (7000 Mol) *N*,*N*,*N*,2,2,6,6-Heptamethylpiperidinyl-4-ammoniumchlorid (abgekürzt als "TMA-TEMP") zugegeben und gelöst. Zum Schluss wurden 803 kg H₂O₂ als 50 Gew.-% wässrige Lösung (entspricht 401.5 kg und somit 11808 Mol H₂O₂) zugegeben. Bezogen auf TMA-TEMP wurden demnach 1.68 molare Äquivalente H₂O₂ eingesetzt. Die Zugabe des H₂O₂ zur Reaktionsmischung erfolgte in einer Geschwindigkeit, dass die Temperatur der Mischung nicht über 60 °C stieg. Nach beendeter Zugabe des H₂O₂ wurde die Reaktionsmischung für eine Stunde bei 60 °C gerührt.

Die Aufarbeitung des erhaltenen *N*,*N*,*N*,2,2,6,6-Heptamethylpiperidin-1-yloxy-4-ammoniumchlorids (abgekürzt als "TMA-TEMPO") erfolgte durch Erhitzen der Reaktionslösung auf 80 °C und Zugabe 50 Gew.-%iger wässriger Natriumhydroxidlösung, um einen pH von 10.6 bis 11 einzustellen und so überschüssiges H₂O₂ zu zerstören.

Nach Zerstörung von H₂O₂ wurde die Reaktionslösung durch langsame Zugabe von 37 Gew.-% HCl auf pH 5 eingestellt.

Im Vergleich zum Stand der Technik (WO 2018/028830 A1; Beispiel Seite 29, Zeilen 4 bis 14) ergeben sich durch das erfindungsgemäße Verfahren überraschende Vorteile:
1) Eine aufwendige Aufarbeitung wie Fällung und Filtration des Reaktionsprodukts TMA-TEMPO ist nicht nötig.
2) Es werden lediglich 1.68 Äquivalente H₂O₂ eingesetzt, während im Stand der Technik zur Umsetzung von 383 mmol TMA-TEMP 1.15 mol H₂O₂ benötigt werden, also 3 Äquivalente. Das erfindungsgemäße Verfahren ist demnach deutlich effizienter.

## Patentansprüche

1. Verfahren zur Herstellung einer wässrigen Elektrolytlösung **L₁** umfassend mindestens eine Verbindung der Formel **(I)** oder **(II)** mit 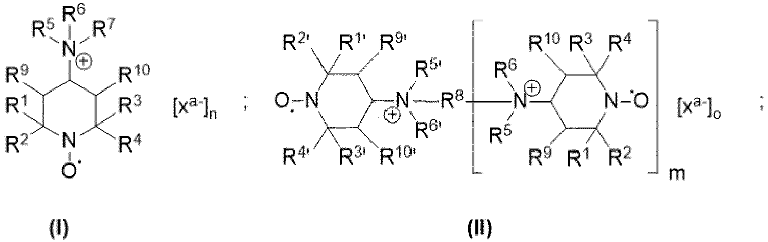
wobei R¹, R², R³, R⁴, R¹', R²', R³', R⁴' unabhängig voneinander jeweils ein C₁-C₆-Alkylrest sind,
wobei R⁹, R¹⁰, R⁹', R¹⁰' unabhängig voneinander jeweils aus der Gruppe bestehend aus Wasserstoff, C₁-C₆-Alkylrest ausgewählt sind,
wobei R⁵, R⁶, R⁵', R⁶' unabhängig voneinander jeweils aus der Gruppe bestehend aus Wasserstoff, Alkyl, Cycloalkyl, Aryl, Aralkyl, Heterocyclyl ausgewählt sind, wobei die Reste Alkyl, Cycloalkyl, Aryl, Aralkyl, Heterocyclyl mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid, Halogen substituiert sein können, wobei außerdem Alkyl durch ein oder mehrere, nicht direkt zueinander benachbarte zweiwertige Reste ausgewählt aus der Gruppe bestehend aus Sauerstoff, Imino unterbrochen sein kann,
wobei außerdem R⁵ und R⁶ zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei außerdem R⁵' und R⁶' zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei R⁷ ein Alkylrest ist, der mit ein oder mehreren Resten ausgewählt aus der Gruppe bestehend aus Alkoxy, Hydroxy, Nitro, Carboxyl, Carboxylester, Carboxylamid, Sulfonat, Sulfonsäureester, Sulfonamid, Halogen substituiert sein kann und der durch ein oder mehrere, nicht direkt zueinander benachbarte zweiwertige Reste ausgewählt aus der Gruppe bestehend aus Sauerstoff, Imino unterbrochen sein kann,
wobei R⁸ eine p-wertige organische Brückengruppe ist, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann, und die durch ein oder mehrere, nicht direkt zueinander benachbarte zweiwertige Reste ausgewählt aus der Gruppe bestehend aus Sauerstoff, Imino unterbrochen sein kann,
X^{a-} ein organisches Anion, ein anorganisches Anion oder eine Mischung dieser Anionen ist,
a die Wertigkeit des Anions X^{a-} angibt und eine ganze Zahl von 1 bis 10.000 bedeutet,
p eine ganze Zahl von 2 bis 6 bedeutet,
m eine ganze Zahl mit dem Wert "p - 1" bedeutet,
n eine Zahl mit dem Wert "1 / a" bedeutet,
o eine Zahl mit dem Wert "(1 + m) / a" bedeutet,
**gekennzeichnet dadurch, dass**
(a) mindestens eine Verbindung der Formel **(III)** oder **(IV)** mit 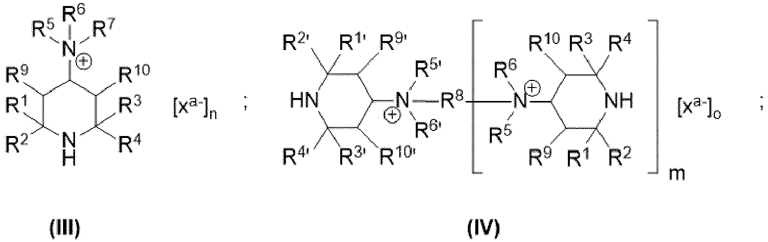 mit H₂O₂ in einer wässrigen Lösung **L₂** umfassend mindestens ein Salz **S,** was aus der Gruppe bestehend aus Hydrogencarbonatsalz, Carbonatsalz ausgewählt ist, zu einer Verbindung der Formel **(I)** bzw. **(II)** umgesetzt wird und
(b) nach der Umsetzung gemäß Schritt (a) H₂O₂, falls dieses dann noch in der wässrigen Lösung **L₂** enthalten ist, aus **L₂** entfernt wird,
wodurch eine wässrige Lösung **L₃** umfassend mindestens eine Verbindung der Formel **(I)** bzw. **(II)** und mindestens ein Salz **S** erhalten wird,
(c) ein saurer pH-Wert in **L₃** eingestellt wird, wodurch das Salz **S** mindestens teilweise aus **L₃** entfernt und die wässrige Elektrolytlösung **L₁** erhalten wird.

2. Verfahren nach Anspruch 1,
wobei R¹, R², R³, R⁴, R¹', R²', R³', R⁴' unabhängig voneinander jeweils ein C₁-C₄-Alkylrest sind,
wobei R⁵, R⁶, R⁵', R⁶' unabhängig voneinander jeweils aus der Gruppe bestehend aus Wasserstoff, Alkyl, wobei Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind,
wobei außerdem R⁵ und R⁶ zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei außerdem R⁵' und R⁶' zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei R⁹, R¹⁰, R⁹', R¹⁰' unabhängig voneinander jeweils aus der Gruppe bestehend aus Wasserstoff, C₁-C₄-Alkylrest ausgewählt sind,
wobei R⁷ C₁-C₆-Alkyl, welches durch ein oder mehrere, nicht direkt zueinander benachbarte zweiwertige Reste ausgewählt aus der Gruppe bestehend aus Sauerstoff, Imino unterbrochen sein kann, ist,
wobei R⁸ eine Alkylengruppe, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann und die durch ein oder mehrere, nicht direkt zueinander benachbarte zweiwertige Reste ausgewählt aus der Gruppe bestehend aus Sauerstoff, Imino unterbrochen sein kann, ist,
X^{a-} ein anorganisches Anion ist,
a bei Verbindungen der Formel **(I)** und **(III)** eine ganze Zahl von 1 bis 3 und bei Verbindungen der Formel **(II)** und **(IV)** eine ganze Zahl von 1 bis 6 bedeutet,
m = 1 bedeutet
n eine Zahl mit dem Wert "1 / a" bedeutet,
o eine Zahl mit dem Wert "2 / a" bedeutet.

3. Verfahren nach Anspruch 2, wobei R¹, R², R³, R⁴, R¹', R²', R³', R⁴' jeweils Methyl sind,
wobei R⁵, R⁶, R⁵', R⁶' unabhängig voneinander jeweils aus der Gruppe bestehend aus Methyl, Ethyl ausgewählt sind,
wobei R⁹, R¹⁰, R⁹', R¹⁰' jeweils Wasserstoff sind,
wobei R⁷ aus der Gruppe bestehend aus Methy, Ethyl ausgewählt ist,
wobei R⁸ eine C₁-C₁₀-Alkylengruppe ist,
X^{a-} aus der Gruppe bestehend aus Halogenidion mit Wertigkeit a = 1, Hydroxyion mit Wertigkeit
a = 1, Phosphation mit Wertigkeit a = 3, Sulfation mit Wertigkeit a = 2, Perchloration mit Wertigkeit
a = 1, Hexafluorophosphation mit Wertigkeit a = 1, Tetrafluoroboration mit Wertigkeit a = 1 ausgewählt ist,
m = 1 bedeutet,
n eine Zahl mit dem Wert "1 / a" bedeutet,
o eine Zahl mit dem Wert "2 / a" bedeutet.

4. Verfahren nach einem der Ansprüche 1 bis 3 zur Herstellung einer wässrigen Lösung **L₁** mindestens einer Verbindung der Formel **(I),** wobei mindestens eine Verbindung der Formel **(III)** eingesetzt wird und die Definitionen der Reste R¹, R², R³, R⁴, R⁵, R⁶, R⁷, R⁹, R¹⁰, X^{a-} und der Variablen n nach einem der Ansprüche 1 bis 3 entsprechend gelten.

5. Verfahren nach einem der Ansprüche 1 bis 4, wobei Schritt (a) bei einem von pH-Wert von 7.5 bis < 10.5 durchgeführt wird.

6. Verfahren nach einem der Ansprüche 1 bis 5, wobei Schritt (a) bei einer Temperatur von 20 °C bis 100 °C und einem Druck von 0.5 bar bis 2 bar durchgeführt wird.

7. Verfahren nach einem der Ansprüche 1 bis 6, wobei das Salz **S** aus der Gruppe bestehend aus Alkalimetallcarbonat, Erdalkalimetallcarbonat, Alkalimetallhydrogencarbonat, Erdalkalimetallhydrogencarbonat ausgewählt ist.

8. Verfahren nach einem der Ansprüche 1 bis 7, wobei das molare Verhältnis der Stoffmenge aller von der wässrigen Lösung **L₂** umfassten Salze S zur gesamten Stoffmenge aller in Schritt (a) eingesetzten Verbindungen der Formeln **(III)** und **(IV)** im Bereich 10 : 1 bis 1 : 1000 liegt.

9. Verfahren nach einem der Ansprüche 1 bis 8, wobei das molare Verhältnis der Gesamtstoffmenge aller in Schritt (a) eingesetzten Verbindungen der Formeln **(III)** und **(IV)** zur Gesamtstoffmenge des in Schritt (a) eingesetzten H₂O₂ im Bereich 9 : 1 bis 1 : 9 liegt.

10. Verfahren nach Anspruch 9, wobei das molare Verhältnis der Gesamtstoffmenge aller in Schritt (a) eingesetzten Verbindungen der Formeln **(III)** und **(IV)** zur Gesamtstoffmenge des in Schritt (a) eingesetzten H₂O₂ im Bereich 1 : 1.05 bis 1 : 9 liegt.

11. Verfahren nach Anspruch 10, wobei in Schritt (b) H₂O₂ durch Einstellen der wässrigen Lösung **L₂** auf einen pH-Wert von ≥ 10.5 entfernt wird.

12. Verfahren nach Anspruch 10, wobei in Schritt (b) H₂O₂ durch Umsetzen der wässrigen Lösung **L₂** in Gegenwart eines Katalysators entfernt wird.

13. Verfahren nach einem der Ansprüche 1 bis 12, wobei der Anteil aller Verbindungen der Formel **(I)** und **(II)** in der wässrigen Elektrolytlösung **L₁** im Bereich 10 bis 80 Gew.-%, bezogen auf das Gesamtgewicht der der wässrigen Elektrolytlösung **L₁,** liegt.

## Claims

1. Process for preparing an aqueous electrolyte solution **L₁** containing at least one compound of the formula **(I)** or **(II)** with 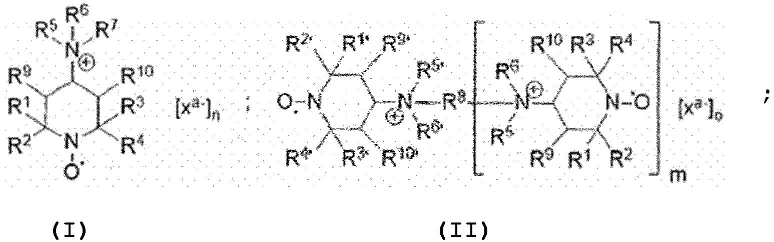
where R¹, R², R³, R⁴, R¹', R²', R³' and R⁴' are each independently a C₁-C₆ alkyl radical,
where R⁹, R¹⁰, R⁹' and R¹⁰' are each independently selected from the group consisting of hydrogen and C₁-C₆ alkyl radical,
where R⁵, R⁶, R⁵' and R⁶' are each independently selected from the group consisting of hydrogen, alkyl, cycloalkyl, aryl, aralkyl and heterocyclyl, where the radicals alkyl, cycloalkyl, aryl, aralkyl and heterocyclyl may be substituted by one or more radicals selected from the group consisting of alkoxy, hydroxy, carboxyl, carboxylic ester, carboxamide, sulfonate, sulfonic ester, sulfonamide and halogen, where in addition alkyl may be interrupted by one or more divalent radicals that are not directly adjacent to one another and are selected from the group consisting of oxygen and imino,
where in addition R⁵ and R⁶ may together form an alkylene group that may be interrupted by one or more divalent radicals that are not directly adjacent to one another and are selected from the group consisting of oxygen and imino,
where in addition R⁵' and R⁶' may together form an alkylene group that may be interrupted by one or more divalent radicals that are not directly adjacent to one another and are selected from the group consisting of oxygen and imino,
where R⁷ is an alkyl radical that may be substituted by one or more radicals selected from the group consisting of alkoxy, hydroxy, nitro, carboxyl, carboxylic ester, carboxamide, sulfonate, sulfonic ester, sulfonamide and halogen and that may be interrupted by one or more divalent radicals that are not directly adjacent to one another and are selected from the group consisting of oxygen and imino,
where R⁸ is a p-valent organic bridging group that may be substituted by one or more radicals selected from the group consisting of alkyl, hydroxy, nitro, carboxyl, carboxylic ester, carboxamide, sulfonate, sulfonic ester, sulfonamide and halogen and that may be interrupted by one or more divalent radicals that are not directly adjacent to one another and are selected from the group consisting of oxygen and imino,
X^{a-} is an organic anion, an inorganic anion or a mixture of these anions,
a denotes the valency of the anion X^{a-} and is an integer from 1 to 10 000,
p is an integer from 2 to 6,
m is an integer with the value of "p - 1",
n is an integer with the value of "1 / a",
o is an integer with the value of "(1 + m) / a",
**characterized in that**
(a) at least one compound of the formula **(III)** or **(IV)** with 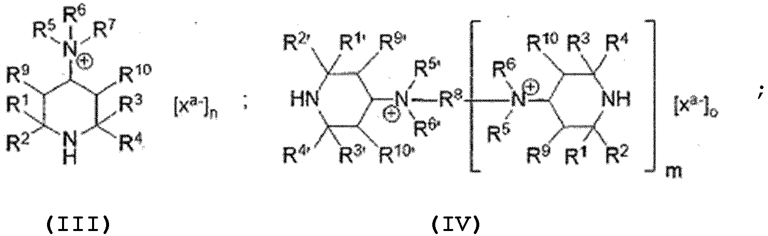 is reacted with H₂O₂ in an aqueous solution **L₂** containing at least one salt **S** selected from the group consisting of hydrogen carbonate salt and carbonate salt to form a compound of the formula **(I)** or **(II)** and
(b) after the reaction according to step (a) H₂O₂, if this is then still present in the aqueous solution **L₂,** is removed from **L₂,**
whereby an aqueous solution **L₃** containing at least one compound of the formula **(I)** or **(II)** and at least one salt **S** is obtained,
(c) an acidic pH is established in **L₃**, whereby the salt **S** is at least partially removed from **L₃** and the aqueous electrolyte solution **L₁** is obtained.

2. Process according to Claim 1,
where R¹, R², R³, R⁴, R¹', R²', R³' and R⁴' are each independently a C₁-C₄ alkyl radical,
where R⁵, R⁶, R⁵' and R⁶' are each independently selected from the group consisting of hydrogen and alkyl, where alkyl may be interrupted by one or more divalent radicals that are not directly adjacent to one another and are selected from the group consisting of oxygen and imino,
where in addition R⁵ and R⁶ may together form an alkylene group that may be interrupted by one or more divalent radicals that are not directly adjacent to one another and are selected from the group consisting of oxygen and imino,
where in addition R⁵' and R⁶' may together form an alkylene group that may be interrupted by one or more divalent radicals that are not directly adjacent to one another and are selected from the group consisting of oxygen and imino,
where R⁹, R¹⁰, R⁹' and R¹⁰' are each independently selected from the group consisting of hydrogen and C₁-C₄ alkyl radical,
where R⁷ is C₁-C₆ alkyl, which may be interrupted by one or more divalent radicals that are not directly adjacent to one another and are selected from the group consisting of oxygen and imino,
where R⁸ is an alkylene group that may be substituted by one or more radicals selected from the group consisting of alkyl, hydroxy, nitro, carboxyl, carboxylic ester, carboxamide, sulfonate, sulfonic ester, sulfonamide and halogen and that may be interrupted by one or more divalent radicals that are not directly adjacent to one another and are selected from the group consisting of oxygen and imino,
X^{a-} is an inorganic anion,
a in compounds of the formula **(I)** and **(III)** is an integer from 1 to 3 and in compounds of the formula **(II)** and **(IV)** is an integer from 1 to 6,
m = 1,
n is an integer with the value of "1 / a",
o is an integer with the value of "2 / a".

3. Process according to Claim 2, where R¹, R², R³, R⁴, R¹', R²', R³' and R⁴' are each methyl,
where R⁵, R⁶, R⁵' and R⁶' are each independently selected from the group consisting of methyl and ethyl,
where R⁹, R¹⁰, R⁹' and R¹⁰' are each hydrogen,
where R⁷ is selected from the group consisting of methyl and ethyl,
where R⁸ is a C₁-C₁₀ alkylene group,
X^{a-} is selected from the group consisting of halide ion with valency a = 1, hydroxy ion with valency a = 1, phosphate ion with valency a = 3, sulfate ion with valency a = 2, perchlorate ion with valency a = 1, hexafluorophosphate ion with valency a = 1, tetrafluoroborate ion with valency a = 1,
m = 1,
n is a number with the value of "1 / a",
o is a number with the value of "2 / a".

4. Process according to any of Claims 1 to 3 for preparing an aqueous solution **L₁** of at least one compound of the formula **(I),** where at least one compound of the formula **(III)** is used and the definitions of the radicals R¹, R², R³, R⁴, R⁵, R⁶, R⁷, R⁹, R¹⁰ and X^{a-} and of the variables n according to any of Claims 1 to 3 apply accordingly.

5. Process according to any of Claims 1 to 4, where step (a) is performed at a pH from 7.5 to < 10.5.

6. Process according to any of Claims 1 to 5, where step (a) is performed at a temperature from 20°C to 100°C and a pressure of 0.5 bar to 2 bar.

7. Process according to any of Claims 1 to 6, where the salt **S** is selected from the group consisting of alkali metal carbonate, alkaline earth metal carbonate, alkali metal hydrogen carbonate and alkaline earth metal hydrogen carbonate.

8. Process according to any of Claims 1 to 7, where the molar ratio of the molar amount of all the salts **S** present in the aqueous solution **L₂** to the total molar amount of all compounds of the formulae **(III)** and **(IV)** used in step (a) is in the range from 10:1 to 1:1000.

9. Process according to any of Claims 1 to 8, where the molar ratio of the total molar amount of all compounds of the formulae **(III)** and **(IV)** used in step (a) to the total molar amount of the H₂O₂ used in step (a) is in the range from 9:1 to 1:9.

10. Process according to Claim 9, where the molar ratio of the total molar amount of all compounds of the formulae **(III)** and **(IV)** used in step (a) to the total molar amount of the H₂O₂ used in step (a) is in the range from 1:1.05 to 1:9.

11. Process according to Claim 10, where in step (b) H₂O₂ is removed by adjusting the aqueous solution **L₂** to a pH of ≥ 10.5.

12. Process according to Claim 10, where in step (b) H₂O₂ is removed by reacting the aqueous solution **L₂** in the presence of a catalyst.

13. Process according to any of Claims 1 to 12, where the proportion of all compounds of the formulae **(I)** and **(II)** in the aqueous electrolyte solution **L₁** is in the range from 10% to 80% by weight based on the total weight of the aqueous electrolyte solution **L₁**.

## Revendications

1. Procédé pour la préparation d'une solution aqueuse électrolytique L₁ comprenant au moins un composé de formule (I) ou (II) 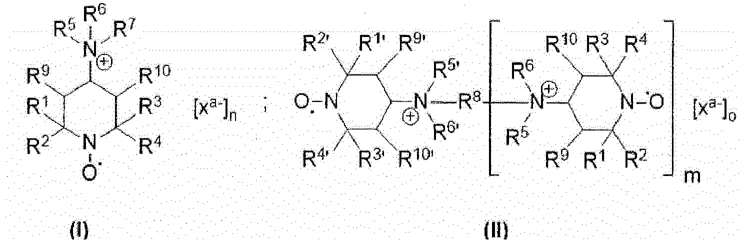
R¹, R², R³, R⁴, R¹', R²', R³', R⁴' représentant, indépendamment les uns des autres, à chaque fois un radical C₁-C₆-alkyle,
R⁹, R¹⁰, R⁹', R¹⁰' étant choisis, indépendamment les uns des autres, à chaque fois dans le groupe constitué par hydrogène, radical C₁-C₆-alkyle,
R⁵, R⁶, R⁵', R⁶' étant choisis, indépendamment les uns des autres, à chaque fois dans le groupe constitué par hydrogène, alkyle, cycloalkyle, aryle, aralkyle, hétérocyclyle ; les radicaux alkyle, cycloalkyle, aryle, aralkyle, hétérocyclyle pouvant être substitués par un ou plusieurs radicaux choisis dans le groupe constitué par alcoxy, hydroxy, carboxyle, ester carboxylique, carboxylamide, sulfonate, ester d'acide sulfonique, sulfonamide, halogène ; alkyle pouvant en outre être interrompu par un ou plusieurs radicaux divalents non directement adjacents choisis dans le groupe constitué par oxygène, imino,
en outre, R⁵ et R⁶ pouvant former conjointement un groupe alkylène qui peut être interrompu par un ou plusieurs radicaux divalents non directement adjacents choisis dans le groupe constitué par oxygène, imino,
en outre, R⁵' et R⁶' pouvant former conjointement un groupe alkylène qui peut être interrompu par un ou plusieurs radicaux divalents non directement adjacents choisis dans le groupe constitué par oxygène, imino,
R⁷ représentant un radical alkyle qui peut être substitué par un ou plusieurs radicaux choisis dans le groupe constitué par alcoxy, hydroxy, nitro, carboxyle, ester carboxylique, carboxylamide, sulfonate, ester d'acide sulfonique, sulfonamide, halogène et qui peut être interrompu par un ou plusieurs radicaux divalents non directement adjacents choisis dans le groupe constitué par oxygène, imino,
R⁸ représentant un groupe pontant organique p-valent, qui peut être substitué par un ou plusieurs radicaux choisis dans le groupe constitué par alkyle, hydroxy, nitro, carboxyle, ester carboxylique, carboxylamide, sulfonate, ester d'acide sulfonique, sulfonamide, halogène et qui peut être interrompu par un ou plusieurs radicaux divalents non directement adjacents choisis dans le groupe constitué par oxygène, imino,
X^{a-} représentant un anion organique, un anion inorganique ou un mélange de ces anions,
a indiquant la valence de l'anion X^{a-} et signifiant un nombre entier de 1 à 10.000,
p signifiant un nombre entier de 2 à 6,
m signifiant un nombre entier présentant la valeur "p-1",
n signifiant un nombre présentant la valeur "1/a",
o signifiant un nombre présentant la valeur "(1+m)/a", **caractérisé en ce que**
(a) au moins un composé de formule (III) ou (IV) 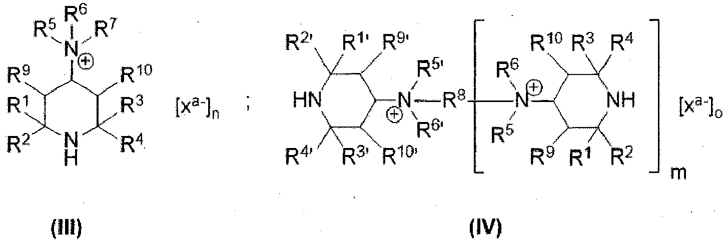 est transformé avec H₂O₂ en une solution aqueuse L₂ comprenant au moins un sel S, qui est choisi dans le groupe constitué par un sel d'hydrogénocarbonate, un sel de carbonate, en un composé de formule (I) ou (II) et
(b) après la transformation selon l'étape (a) le H₂O₂, si celui-ci est encore contenu dans la solution aqueuse L₂, est éliminé de L₂,
suite à quoi on obtient une solution aqueuse L₃ comprenant au moins un composé de formule (I) ou (II) et au moins un sel S,
(c) un pH acide est réglé dans L₃, suite à quoi le sel S est éliminé au moins partiellement de L₃ et la solution aqueuse électrolytique L₁ est obtenue.

2. Procédé selon la revendication 1, R¹, R², R³, R⁴, R¹', R²', R³', R⁴' représentant, indépendamment les uns des autres, à chaque fois un radical C₁-C₄-alkyle,
R⁵, R⁶, R⁵', R⁶' étant choisis, indépendamment les uns des autres, à chaque fois dans le groupe constitué par hydrogène, alkyle ; alkyle pouvant être interrompu par un ou plusieurs radicaux divalents non directement adjacents choisis dans le groupe constitué par oxygène, imino,
en outre, R⁵ et R⁶ pouvant former conjointement un groupe alkylène qui peut être interrompu par un ou plusieurs radicaux divalents non directement adjacents choisis dans le groupe constitué par oxygène, imino,
en outre, R⁵' et R⁶' pouvant former conjointement un groupe alkylène qui peut être interrompu par un ou plusieurs radicaux divalents non directement adjacents choisis dans le groupe constitué par oxygène, imino,
R⁹, R¹⁰, R⁹', R¹⁰' étant choisis, indépendamment les uns des autres, à chaque fois dans le groupe constitué par hydrogène, radical C₁-C₄-alkyle,
R⁷ représentant C₁-C₆-alkyle qui peut être interrompu par un ou plusieurs radicaux divalents non directement adjacents choisis dans le groupe constitué par oxygène, imino,
R⁸ représentant un groupe alkylène, qui peut être substitué par un ou plusieurs radicaux choisis dans le groupe constitué par alkyle, hydroxy, nitro, carboxyle, ester carboxylique, carboxylamide, sulfonate, ester d'acide sulfonique, sulfonamide, halogène et qui peut être interrompu par un ou plusieurs radicaux divalents non directement adjacents choisis dans le groupe constitué par oxygène, imino,
X^{a-} représentant un anion inorganique,
a dans les composés de formule (I) et (III), signifiant un nombre entier de 1 à 3 et, dans les composés de formule (II) et (IV), signifiant un nombre entier de 1 à 6,
m = 1
n signifiant un nombre présentant la valeur "1/a",
o signifiant un nombre présentant la valeur "2/a".

3. Procédé selon la revendication 2, R¹, R², R³, R⁴, R¹',
R²', R³', R⁴' représentant à chaque fois méthyle,
R⁵, R⁶, R⁵', R⁶', étant choisis, indépendamment les uns des autres, à chaque fois dans le groupe constitué par méthyle, éthyle
R⁹, R¹⁰, R⁹', R¹⁰' représentant à chaque fois hydrogène,
R⁷ étant choisi dans le groupe constitué par méthyle, éthyle,
R⁸ représentant un groupe C₁-C₁₀-alkylène,
X^{a-} étant choisi dans le groupe constitué par un ion halogénure présentant la valence a = 1, un ion hydroxy présentant la valence a = 1, un ion phosphate présentant la valence a = 3, un ion sulfate présentant la valence a = 2, un ion perchlorate présentant la valence a = 1, un ion hexafluorophosphate présentant la valence a = 1, un ion tétrafluoroborate présentant la valence a = 1,
m = 1,
n signifiant un nombre présentant la valeur "1/a",
o signifiant un nombre présentant la valeur "2/a".

4. Procédé selon l'une quelconque des revendications 1 à 3 pour la préparation d'une solution aqueuse L₁ d'au moins un composé de formule (I), au moins un composé de formule (III) étant utilisé et les définitions des radicaux R¹, R², R³, R⁴, R⁵, R⁶, R⁷, R⁹, R¹⁰, X^{a-} et des variables n selon l'une quelconque des revendications 1 à 3 s'appliquant de manière correspondante.

5. Procédé selon l'une quelconque des revendications 1 à 4, l'étape (a) étant réalisée à un pH de 7,5 à < 10,5.

6. Procédé selon l'une quelconque des revendications 1 à 5, l'étape (a) étant réalisée à une température de 20°C à 100°C et à une pression de 0,5 bar à 2 bars.

7. Procédé selon l'une quelconque des revendications 1 à 6, le sel S étant choisi dans le groupe constitué par carbonate de métal alcalin, carbonate de métal alcalino-terreux, hydrogénocarbonate de métal alcalin, hydrogénocarbonate de métal alcalino-terreux.

8. Procédé selon l'une quelconque des revendications 1 à 7, le rapport molaire de la quantité de tous les sels S compris dans la solution aqueuse L₂ à la quantité totale de tous les composés des formules (III) et (IV) utilisés dans l'étape (a) se situant dans la plage de 10:1 à 1:1000.

9. Procédé selon l'une quelconque des revendications 1 à 8, le rapport molaire de la quantité totale de tous les composés des formules (III) et (IV) utilisés dans l'étape (a) à la quantité totale du H₂O₂ utilisé dans l'étape (a) se situant dans la plage de 9:1 à 1:9.

10. Procédé selon la revendication 9, le rapport molaire de la quantité totale de tous les composés des formules (III) et (IV) utilisés dans l'étape (a) à la quantité totale du H₂O₂ utilisé dans l'étape (a) se situant dans la plage de 1:1,05 à 1:9.

11. Procédé selon la revendication 10, dans lequel, dans l'étape (b), le H₂O₂ est éliminé par réglage de la solution aqueuse L₂ à un pH de 10,5.

12. Procédé selon la revendication 10, dans lequel, dans l'étape (b), le H₂O₂ est éliminé par transformation de la solution aqueuse L₂ en présence d'un catalyseur.

13. Procédé selon l'une quelconque des revendications 1 à 12, la proportion de tous les composés de formule (I) et (II) dans la solution aqueuse électrolytique L₁ se situant dans la plage de 10 à 80% en poids, par rapport au poids total de la solution aqueuse électrolytique L₂.
